(19) Europäisches Patentamt
European Patent Office
Office européen des brevets

(11) **EP 3 007 127 A1**

(12) **EUROPEAN PATENT APPLICATION**
published in accordance with Art. 153(4) EPC

(43) Date of publication:
**13.04.2016 Bulletin 2016/15**

(51) Int Cl.:
***G06Q 50/22*** *(2012.01)*

(21) Application number: **14801361.8**

(22) Date of filing: **17.04.2014**

(86) International application number:
**PCT/JP2014/002175**

(87) International publication number:
**WO 2014/188657 (27.11.2014 Gazette 2014/48)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **24.05.2013 JP 2013110298**

(71) Applicant: **Seiko Epson Corporation
Shinjuku-ku
Tokyo 163-0811 (JP)**

(72) Inventors:
• **KATO, Hidetada
Suwa-shi
Nagano 392-8502 (JP)**
• **IKEJIRI, Masahisa
Suwa-shi
Nagano 392-8502 (JP)**
• **SANO, Megumi
Suwa-shi
Nagano 392-8502 (JP)**

(74) Representative: **Hoffmann Eitle
Patent- und Rechtsanwälte PartmbB
Arabellastraße 30
81925 München (DE)**

(54) **INFORMATION PROCESSING SYSTEM, PROGRAM, AND INFORMATION PROCESSING METHOD**

(57) To provide an information processing system, a program, an information processing method, and the like that make it easy to reduce an exercise risk and maintain motivation by creating advice information using staying time information representing a staying time in a fat burning zone.

An information processing system 100 includes an information acquiring unit 110 that performs acquisition processing for information, a processing unit 120 that performs processing on the basis of the acquired information, and an output processing unit 130 that performs output processing for information created by the processing unit 120. The information acquiring unit 110 performs the acquisition processing for at least two kinds of information among staying time information, body information, and target information concerning a weight loss. The processing unit 120 creates advice information concerning the weight loss on the basis of a relational expression representing a relation between fluctuation in body weight or a body fat amount and the staying time information and the at least two kinds of information. The output processing unit 130 performs the output processing for the advice information.

FIG. 2

**Description**

Technical Field

The present invention relates to an information processing system, a program, an information processing method, and the like.

Background Art

In recent years, according to an increase in interest in health care, services and systems for supporting health maintenance and improvement are widely used. Various methods are conceivable as methods of such services and systems. A method of using information of an individual user, who is a subject, is also used taking into account, for example, improvement of accuracy of advice information provided by the services and the like.

For example, PTL 1 discloses a method of displaying, using an energy increase and decrease amount, a prediction of a body weight change, duration to a predicted point in time, and an energy increase and decrease amount during the duration. The energy increase and decrease amount in PTL 1 is information that takes into account both of intake energy and consumed energy. The energy increase and decrease amount is acquired on the basis of information from a sensor such as a physical activity meter worn by a user, meal information input by the user, and the like.

Citation List

Patent Literature

PTL 1: JP-A-2008-33909

Summary of Invention

Technical Problem

In the method of PTL 1, the advice information is, for example, "If the energy increase and decrease amount is reduced x kcal per day, the weight will be z kg after y months". The advice information is based on an energy increase and decrease amount in a given period. When the user receiving the advice attempts to realize a decrease in the energy amount through exercise, it is not easy to learn to which degree an exercise load should be set.

Therefore, the possibility cannot be denied that the user performs exercise with an excessively high load to cause injury or poor health. If the user plans to perform exercise with a high load, it is difficult to continue the exercise for a long period, leading to a decrease in motivation. As a result, it is difficult to stably follow an advice for a long period. It is likely that the health maintenance and improvement, which is the original purpose of the services and the system, cannot be realized.

According to several aspects of the present invention, it is possible to provide an information processing system, a program, an information processing method, and the like that make it easy to reduce an exercise risk and maintain motivation by creating advice information using staying time information representing a staying time in a fat burning zone.

One aspect of the present invention relates to an information processing system including: an information acquiring unit that performs acquisition processing for information; a processing unit that performs processing on the basis of the information acquired by the information acquiring unit; and an output processing unit that performs output processing for the information created by the processing unit. The information acquiring unit performs the acquisition processing for at least two kinds of information among staying time information representing time in which a value represented by pulse wave information of a user is a value within a given fat burning zone, body information of the user, and target information concerning a weight loss of the user. The processing unit performs processing for creating advice information concerning the weight loss on the basis of a relational expression representing a relation between fluctuation in body weight or a body fat amount of the user and the staying time information and the at least two kinds of information acquired by the information acquiring unit. The output processing unit performs the output processing for the advice information created by the processing unit.

In the one aspect of the present invention, the advice information is generated using the relational expression between the fluctuation in the body weight or the body fat amount and the staying time information and the at least two kinds of information among the staying time information, the body information, and the target information. Therefore, since the staying time information is used, it is possible to, for example, suppress implementation of exercise with an excessively high load and efficiently perform fat burning by exercise. By deciding the relational expression, it is possible to, for example, easily associate the staying time information and the fluctuation in the body weight or the like.

In the one aspect of the present invention, when a value corresponding to an integrated value of the staying time information in a given period is represented as T_zone, a fluctuation amount of the body weight or the body fat amount of the user in the given period is represented as W, and a fat burning coefficient is represented as Kfat, the relational expression may be T_zone×Kfat=W.

Consequently, it is possible to associate the staying time information and the fluctuation in the body weight or the like using the relational expression.

In the one aspect of the present invention, the information acquiring unit may perform the acquisition processing for the staying time information and the body information. The processing unit may perform processing for creating the advice information concerning an intake calorie of the user on the basis of the staying time information, the body information, and the relational expression.

Consequently, it is possible to generate the advice information concerning the intake calorie from the staying time information, the body information, and the relational expression.

In the one aspect of the present invention, the information acquiring unit may perform the acquisition processing for the staying time information up to given advice timing within an advice period and perform the acquisition processing for acquiring, as the body information, a measured value of the body weight or the body fact amount of the user up to the advice timing. The processing unit may perform processing for calculating an estimated value of the body weight or the body fat amount at the advice timing corresponding to the staying time information on the basis of the staying time information and the relational expression and creating the advice information concerning the intake calorie on the basis of comparison processing of the calculated estimated value and the measured value.

Consequently, it is possible to acquire a measured value of the staying time information up to the advice timing and acquire the measured value of the body weight or the body fact amount as the body information. It is also possible to create the advice information concerning the intake calorie as the advice information concerning the weight loss on the basis of the comparison processing.

In the one aspect of the present invention, the information acquiring unit may perform the acquisition processing for the staying time information and the target information. The processing unit may perform processing for creating the advice information concerning an exercise amount of the user on the basis of the staying time information, the target information, and the relational expression.

Consequently, it is possible to generate the advice information concerning the exercise amount from the staying time information, the target information, and the relational expression.

In the one aspect of the present invention, the information acquiring unit may perform the acquisition processing for the staying time information up to given advice timing within an advice period and perform the acquisition processing for acquiring, as the target information, a target value of the body weight or the body fat amount of the user at the advice timing set before the advice timing. The processing unit may perform processing for calculating an estimated value of the body weight or the body fat amount corresponding to the staying time information on the basis of the staying time information and the relational expression and creating the advice information concerning the exercise amount on the basis of comparison processing of the calculated estimated value and the target value.

Consequently, it is possible to acquire a measured value of the staying time information up to the advice timing and acquire, as the target information, the target value of the body weight or the body fat amount corresponding to the advice timing set beforehand. It is also possible to create the advice information concerning the exercise amount as the advice information concerning a weight loss on the basis of the comparison processing.

In the one aspect of the present invention, the information acquiring unit may perform the acquisition processing for acquiring, as the body information, a present value of the body weight or the body fat amount of the user at the advice timing and perform the acquisition processing for acquiring, as the target information, a target value of the body weight or the body fact amount of the user and a target period representing a period until the target value is attained. The processing unit may perform processing for calculating, on the basis of the present value, the target value, the target period, and the relational expression, the staying time information necessary for attaining the target value within the target period.

Consequently, it is possible to acquire the present value of the body weight or the body fat amount at the advice timing as the body information and acquire the target value of the body weight or the body fat amount and the target period as the target information. It is possible to, for example, calculate, on the basis of the relational expression, at the advice timing, the staying time information necessary for attaining a target.

In the one aspect of the present invention, the information acquiring unit may perform the acquisition processing for the body information and the target information. The processing unit may perform processing for calculating, on the basis of the body information, the target information, and the relational expression, the staying time information necessary for attaining a target represented by the target information.

Consequently, it is possible to calculate, from the body information, the target information, and the relational expression, the staying time information necessary for attaining the target.

In the one aspect of the present invention, the information acquiring unit may perform the acquisition processing for acquiring a present value of the body weight or the body fat amount of the user as the body information and perform the acquisition processing for acquiring, as the target information, a target value of the body weight or the body fat amount of the user and a target period representing a period until the target value is attained. The processing unit may perform processing for calculating, on the basis of the present value, the target value, the target period, and the relational expression, the staying time information necessary for attaining the target value within the target period.

Consequently, it is possible to acquire the present value of the body weight or the body fat amount as the body information and acquire the target value of the body weight or the body fat amount and the target period as the target information. It is possible to, for example, calculate, on the basis of the relational expression, the staying time information necessary for attaining the target.

In the one aspect of the present invention, the processing unit may perform processing for generating correction instruction information for instructing correction of at least one of the target value and the target period when a staying time represented by the calculated staying time information is larger than a given threshold. When the information acquiring unit performs the acquisition processing for at least one of the target value and the target period corrected on the basis of the correction instruction information, the processing unit may perform processing for calculating again, on the basis of at least one of the target value and the target period after the correction, the staying time information necessary for attaining the target value within the target period.

Consequently, when the staying time information necessary for attaining the target is an unreasonable value, it is possible to perform the correction processing for the target. Therefore, it is possible to perform reasonable target setting and the like.

In the one aspect of the present invention, at start timing of an advice period, the information acquiring unit may perform the acquisition processing for the body information and the target information. The processing unit may perform processing for calculating, on the basis of the body information, the target information, and the relational expression, the staying time information necessary for attaining a target represented by the target information.

Consequently, it is possible to, for example, perform target setting processing at the start timing of the advice period.

Another aspect of the present invention relates to a program for causing a computer to function as: an information acquiring unit that performs acquisition processing for information; a processing unit that performs processing on the basis of the information acquired by the information acquiring unit; and an output processing unit that performs output processing for the information created by the processing unit. The information acquiring unit performs the acquisition processing for at least two kinds of information among staying time information representing time in which a value represented by pulse wave information of a user is a value within a given fat burning zone, body information of the user, and target information concerning a weight loss of the user. The processing unit performs processing for creating advice information concerning the weight loss on the basis of a relational expression representing a relation between fluctuation in body weight or a body fat amount of the user and the staying time information and the at least two kinds of information acquired by the information acquiring unit. The output processing unit performs the output processing for the advice information created by the processing unit.

Another aspect of the present invention is an information processing method for causing a computer to execute: acquisition processing for at least two kinds of information among staying time information representing time in which a value represented by pulse wave information of a user is a value within a given fat burning zone, body information of the user, and target information concerning a weight loss of the user; processing for creating advice information concerning the weight loss on the basis of a relational expression representing a relation between fluctuation in body weight or a body fat amount of the user and the staying time information and the at least two kinds of information acquired by the acquisition processing; and the output processing for the created advice information.

Brief Description of the Drawings

[Fig. 1] Fig. 1 is a diagram for explaining a data flow of an embodiment.
[Fig. 2] Fig. 2 is a configuration example of an information processing system of the embodiment.
[Fig. 3] Fig. 3 is a configuration example of the information processing system realized by a server system.
[Fig. 4] Fig. 4 is a configuration example of the information processing system is realized by a smart phone.
[Fig. 5] Fig. 5 is a relation chart of a cumulative value of a zone staying time and a cumulative value of a fat burning amount.
[Fig. 6] Fig. 6 is a relation chart between a zone staying rate and a fat burning amount per unit time.
[Fig. 7] Fig. 7 is a diagram showing a relation between the zone staying rate and the fat burning amount per unit time and a relation between the zone staying rate and a body fat decrease amount per unit time.
[Fig. 8] Fig. 8 is a relation chart between the body fat decrease amount and a body weight decrease amount.

[Fig. 9] Fig. 9(A) to Fig. 9(C) are specific examples of display images for displaying advice information.

[Fig. 10] Fig. 10 is an explanatory diagram of a prediction line, a regression line, and a target line of a body weight decrease.

[Fig. 11] Fig. 11 is a relation chart between a difference between a measured value and a predicted value of the body fat decrease amount and an intake calorie amount.

[Fig. 12] Fig. 12 is an explanatory diagram of comparison processing for the prediction line and the regression line of the body weight decrease.

[Fig. 13] Fig. 13 is an explanatory diagram of comparison processing for the prediction line and the target line of the body weight decrease.

[Fig. 14] Fig. 14 is a diagram for explaining resetting processing for a target.

[Fig. 15] Fig. 15 is another diagram for explaining the resetting processing for a target.

[Fig. 16] Fig. 16 is a diagram for explaining processing at an advice period end time.

Description of Embodiments

An embodiment is explained below. Note that the embodiment explained below does not unduly limit contents of the present invention described in claims. Not all of components explained in the embodiment are essential constituent elements of the present invention.

1. Method of this embodiment

First, a method of this embodiment is explained. In recent years, according to an increase in social health consciousness, services and the like for health maintenance and improvement are widely provided. For example, since the term "metabolic syndrome" is widely known concerning obesity, there are many users who periodically measure values of body weight and an abdominal circumference. However, even if only measured values are acquired, for the users, who are not medical experts, it is difficult to appropriately reflect fluctuation and the like of the measured values on lifestyles and the like. Ideally, it is desirable to receive advices by experts such as doctors. However, when problems such as burdens on experts, who take care of a large number of subjects, and costs are taken into account, there is an increasing request for a system that automates generation of advice information to some extent.

Various factors such as exercise and meals are conceivable concerning fluctuation of body weight and the like. Therefore, it is important in generating appropriate advice information to acquire an exercise state and a condition of meals of a user. Concerning the exercise state and the like, by attaching a sensor to the user, it is possible to perform an analysis based on sensor information from the sensor. For example, if a pedometer (in a broad sense, a physical activity meter) is realized by an acceleration sensor or the like, it is possible to estimate an exercise amount of the user from information such as the number of steps.

As a conventional method that takes into account the above, PTL 1 discloses an advice generating method based on an energy increase and decrease amount. In PTL 1, a consumed energy amount by exercise and the like and an intake energy amount by meals and the like are acquired by some method. The energy increase and decrease amount is calculated using both of the consumed energy amount and the intake energy amount. Advice information is generated on the basis of a calculation logic that associates the energy increase or decrease amount and body weight fluctuation.

The advice information in PTL 1 is, for example, "If the energy increase and decrease amount is reduced x kcal per day, the weight will be z kg after y months". When the user receiving the advice attempts to realize a decrease in the energy amount through exercise, it is not easy to learn to which degree an exercise load should be set.

Specifically, it is unclear with which load exercise should be performed for which degree of time to realize energy consumption of x kcal. Therefore, the user needs to refer to some information. Whereas the same degree of energy consumption is realized in a short time in exercise with a high load and it is necessary to continue exercise with a low load for a long time compared with the exercise with the high load, in the method of PTL 1, only the energy increase and decrease amount is advised. That is, to which degree an exercise load is set is left to determination of the user. Therefore, it is likely that injury or poor health is caused by performing exercise with an excessively high load. Whereas it is known that efficiency of fat burning is different depending on a load of exercise, it is likely that the user performs exercise with a load inefficient for fat burning. In that case, a sufficient health maintenance and improvement effect is not obtained for an effort of the user. Further, for a user who plans to perform exercise with a high load and a user who cannot efficiently reduce body weight although trying to perform exercise, it is highly likely that motivation for a service use decreases. As a result, a continuous service use is not performed. It is difficult to attain the purpose of the service, that is, health maintenance and improvement.

Therefore, the applicant proposes a method of generating advice information on the basis of a zone staying time (in a broad sense, staying time information). The zone staying time is calculated from pulse wave information of the user. For example, when a pulse rate is acquired as the pulse wave information, a given numerical value range of the pulse rate only has to be set as a fat burning zone. Time in which a pulse rate of the user is within the fat burning zone only has to be set as the zone staying time. A value of the pulse count has a correlation with an exercise state of the user. In general, the pulse rate is higher as an exercise load is higher. That is, when it is considered to present a standard value of the zone staying time as advice information, it is possible to suppress exercise with a high load that increases the pulse rate to exceed the fat burning zone. If a numerical value range efficient for fat burning just as indicated by the name is set as the fat burning zone, realizing a predetermined zone staying time corresponds to executing, for a predetermined time, exercise that can efficiently execute fat burning. Therefore, it is possible to cope with the problem.

If a method of displaying the present pulse rate together with the numerical value range of the fat burning zone or displaying, as an icon or the like, information concerning whether the present pulse rate is lower than a lower limit of the fat burning zone, within the zone, or higher than an upper limit of the zone, the user can easily adjust a load of exercise looking at the display. Specifically, if the pulse rate is low with respect to the zone, the user only has to take measures for, for example, increasing a pace of running to increase the exercise load. If the pulse rate is high with respect to the zone, the user only has to reduce the exercise load. That is, by using the idea of the fat burning zone and the zone staying time, the adjustment by the user is easy compared with when an instruction "Please perform exercise of some kcal" is given.

In the following explanation, a system configuration example of an information processing system or the like that generates advice information based on staying time information is explained. Thereafter, it is explained using figures that the staying time information is an appropriate index value in the generation of the advice information. A relational expression between the staying time information and body weight fluctuation is set. Then, specific advice information generation processing using the relational expression is explained.

2. System configuration example

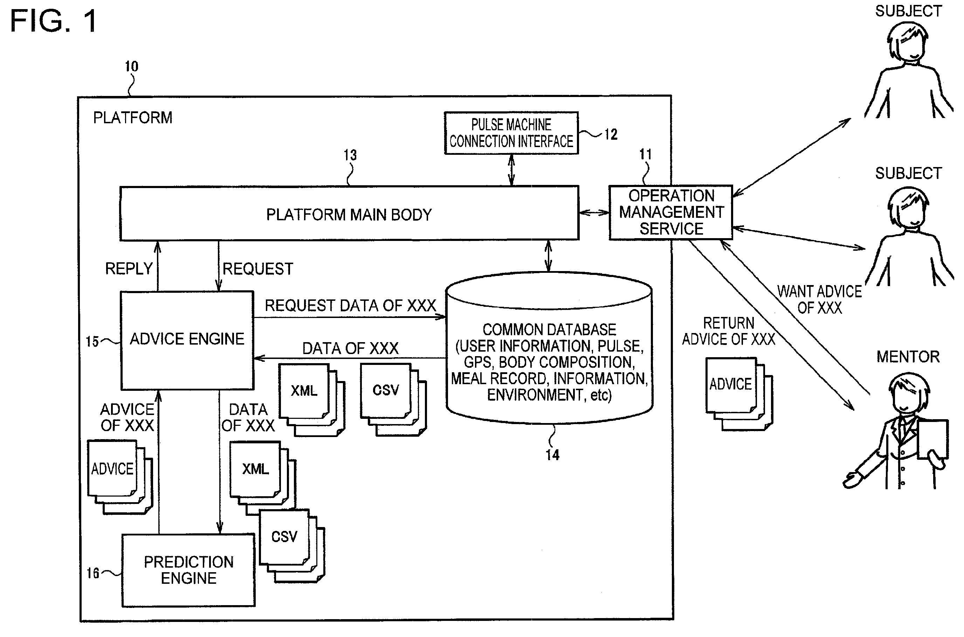

An example of a data flow in a method of this embodiment is shown in Fig. 1. Subjects in Fig. 1 represent users who receive advices for the purpose of health maintenance and improvement. A mentor acts as an adviser to the subjects and plays a role of giving appropriate advices to the subjects. The method of this embodiment does not prevent a form in which the subjects directly refer to advice information generated by the information processing system. However, in the example shown in Fig. 1, the method of this embodiment assumes a form in which advice information is output to the mentor first and the mentor gives advices to the subjects on the basis of the advice information.

As shown in Fig. 1, a platform 10 includes an operation management service 11, a pulse machine connection interface 12, a platform main body 13, a common database 14, an advice engine 15, and a prediction engine 16. For example, the entire platform 10 corresponds to the information processing system of this embodiment.

The operation management service 11 functions as an interface between the platform 10 and the subj ects or the mentor. The operation management service 11 receives pulse wave information, information concerning meals, body information, target information, and the like from the subjects. The operation management service 11 receives a request for data of a specific subject and specific advice information from the mentor and returns information in response to the request.

The pulse machine connection interface 12 is an interface that connects pulse machines (in a narrow sense, pulse meters) worn by the subj ects . The pulse wave information may be acquired from the pulse machines connected to the pulse machine connection interface 12. However, a form in which a machine is not directly connected, for example, the platform 10 is realized as a server system is highly likely. The pulse machine connection interface 12 and the operation management service 11 may be integrally configured. The pulse machine connection interface 12 may be provided on the user (the subject or mentor) side.

The platform main body 13 is connected to the other units of the platform 10, requests acquisition and output of information, information management, or given processing, and performs, for example, acquisition of a response to the request.

The common database 14 stores, as shown in Fig. 1, various kinds of information including individual information such as sex, age, and height, pulse wave information, body information, and target information of the users.

The advice engine 15 generates advice information when an advice for a specific user is requested. Note that, in processing in the advice engine, not only information present at the present point is directly used but also future prediction information estimated from the information is used. The prediction engine 16 performs processing for acquiring the information at the present point and returning the prediction information as a reply. Alternatively, as shown in Fig. 1, advice information based on the prediction information may be created in the prediction engine 16. In that case, the advice engine 15 generates advice information in which the prediction information is not used, that is, advice information based on a measured value or the like and acquires, from the prediction engine 16, advice information in which the prediction information is used.

In the example shown in Fig. 1, first, the subjects use the operation management service 11 as an interface and periodically transmit information such as pulse wave information to the platform 10. When the mentor in charge of a subject XXX needs to give an advice to the subject XXX, the mentor uses the operation management service 11 as an interface and requests the platform 10 to transmit an advice for XXX.

The platform main body 13 receives the request from the mentor and requests the advice engine 15 to transmit advice information for XXX. The advice engine 15 acquires data concerning XXX from the common database 14 in order to generate advice information for XXX. At the same time, the advice engine 15 transmits the acquired data of XXX to the prediction engine 16. The prediction engine 16 performs prediction processing on the basis of the data of XXX and returns prediction information, which is a result of the prediction processing, to the advice engine 15. Details of the prediction processing are explained below. The prediction processing is, for example, processing performed using Expression (5) below.

The advice engine 15 generates advice information on the basis of the data of XXX and the prediction information returned by the prediction engine 16. The generated advice information is transmitted to the mentor via the platform main body 13 and the operation management service 11.

The mentor gives an advice to the subject on the basis of the advice information returned as a response to the advice request. Note that how the advice information is used for an actual advice is left to discretion of the mentor. However, in this embodiment, because appropriate advice information is generated using the zone staying time, it is expected that correction work or the like by the mentor is not large. Therefore, it is possible to reduce labor and time required for an advice of the mentor and suppress fluctuation or the like of advices among mentors corresponding to proficiency levels of the mentors.

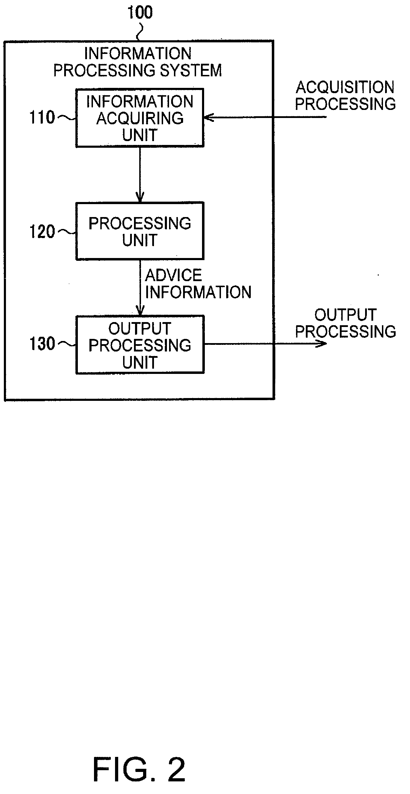

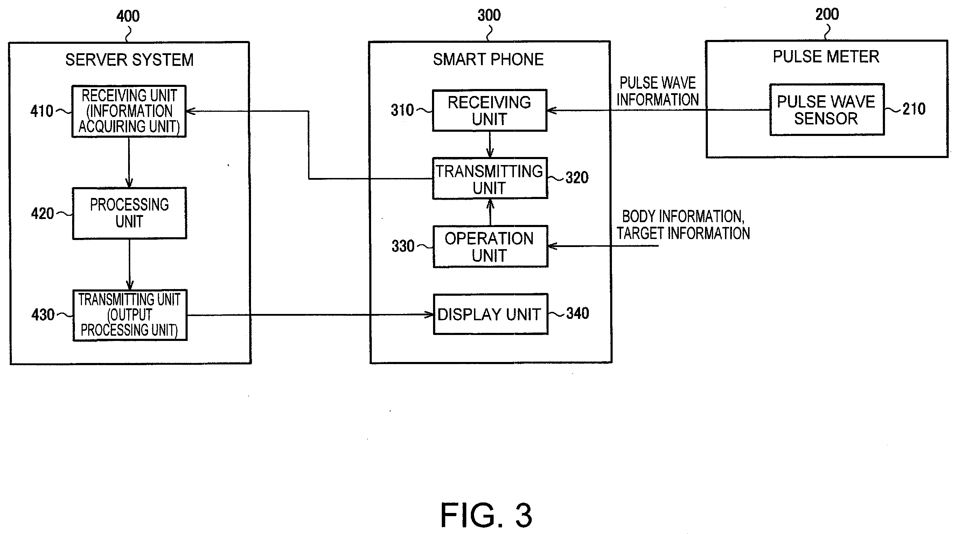

A configuration example of the information processing system is explained with reference to Fig. 2. As shown in Fig. 2, an information processing system 100 includes an information acquiring unit 110, a processing unit 120, and an output processing unit 130. However, the information processing system is not limited to the configuration shown in Fig. 2. Various modified implementations such as omission of a part of components and addition of other components are possible. Various modified implementations are also possible in Fig. 3 and the like.

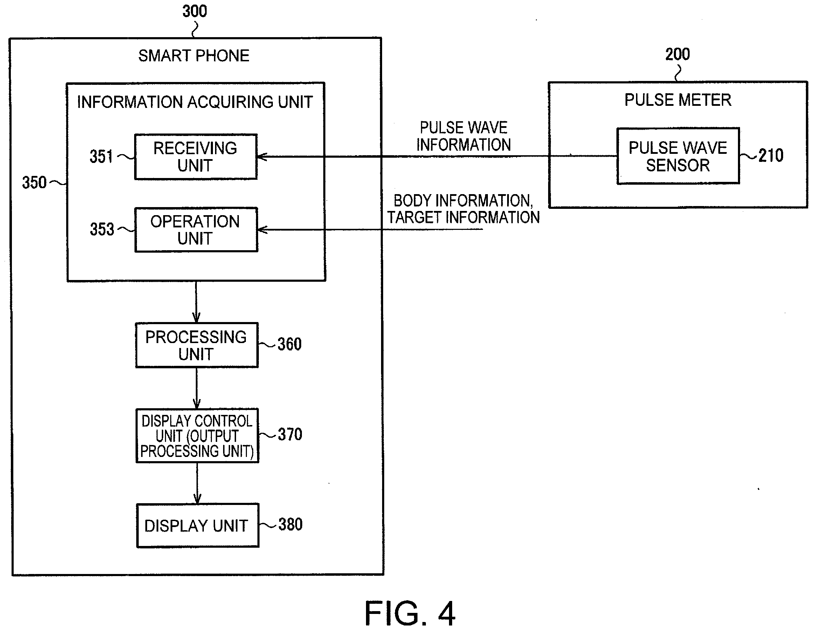

The information acquiring unit 110 performs acquisition processing for various kinds of information. The various kinds of information are staying time information representing time in which a value of a pulse rate is a value within a fat burning zone, body information concerning body weight, abodyfatamount, or the like of the user, target information concerning a weight loss of the user, and the like. Note that, as explained below with reference to Fig. 3 and Fig. 4, the information processing system of this embodiment can be realized by various forms. Therefore, specific content of the acquisition processing for information may be different depending on implementation forms. The acquisition processing in the information acquiring unit 110 may be, for example, reception processing via a network or the like as shown in Fig. 3 or may be reception processing for an information input via an interface as indicated by an operation unit 353 in Fig. 4.

The processing unit 120 generates advice information on the basis of the information acquired by the information acquiring unit 110 and a relational expression. The relational expression is an expression for associating a zone staying time and body weight fluctuation (or fluctuation inabody fat amount). Details of the relational expression are explained below. Details of a specific example of the advice information are also explained below.

The output processing unit 130 performs output processing for the advice information generated by the processing unit 120. Like the acquisition processing in the information acquiring unit 110, the output processing in the output processing unit 130 can be realized by various forms. For example, the output processing may be transmission processing via a network or the like as shown in Fig. 3 or may be processing for performing display control ina displayunit 380 as indicated by a display control unit 370 in Fig. 4.

Specific realization examples of the information processing system are explained with reference to Fig. 3 and Fig. 4. Note that realization examples of the information processing system are not limited to Fig. 3 and Fig. 4. Various modified implementations are possible.

Fig. 3 is an example in which the information processing system 100 is realized as a server system 400. In the example shown in Fig. 3 , the subject wears a pulse meter 200 and carries a smart phone 300. A pulse wave sensor 210 included in the pulse meter 200 transmits pulse wave information to a receiving unit 310 of the smart phone 300. An operation unit 330 of the smart phone 300 receives an input of body information and target information by the subject. A transmitting unit 320 of the smart phone 300 transmits the pulse wave information, the body information, the target information, and the like to the server system 400.

A receiving unit 410 of the server system 400 receives the transmitted pulse wave information and the like. A processing unit 420 applies processing to the information received by the receiving unit 410 and generates advice information. The generated advice information is transmitted to the smart phone 300 from a transmitting unit 430 of the server system 400 and displayed on a display unit 340 of the smart phone 300.

That is, in the example shown in Fig. 3, the receiving unit 410 corresponds to the information acquiring unit 110, the processing unit 420 corresponds to the processing unit 120, and the transmitting unit 430 corresponds to the output processing unit 130. Note that, when the smart phone 300 is carried by the subject in Fig. 3, the advice information is provided to the subject not via the mentor. Therefore, the example is different from the first embodiment in that regard.

Fig. 4 is an example in which the information processing system 100 is realized as the smart phone 300. In the example shown in Fig. 4, the subj ect wears the pulse meter 200 and carries the smart phone 300. The pulse wave sensor 210 included in the pulse meter 200 transmits pulse wave information to a receiving unit 351 included in an information acquiring unit 350 of the smart phone 300. The operation unit 353 included in the information acquiring unit 350 of the smart phone 300 receives an input of body information and target information by the subject.

A processing unit 360 applies processing to the information acquired by the information acquiring unit 350 and generates advice information. A display control unit 370 performs display control of the advice information. A display unit 380 displays advice information according to the control by the display control unit 370.

That is, in the example shown in Fig. 4, the information acquiring unit 350 including the receiving unit 351 and the operation unit 353 corresponds to the information acquiring unit 110 shown in Fig. 2, the processing unit 360 corresponds to the processing unit 120, and the display control unit 370 corresponds to the output processing unit 130.

Note that, in the above explanation, the output from the pulse meter 200 is the pulse wave information. In this case, it is conceivable that, for example, processing for calculating staying time information on the basis of the pulse wave information is performed in a block corresponding to the information acquiring unit 110 in the smart phone 300 or the server system 400. However, this embodiment is not limited to such a configuration. Arithmetic processing for the staying time information may be performed in the pulse meter 200. In that case, firmware or the like of the pulse meter 200 calculates the staying time information on the basis of the pulse wave information from the pulse wave sensor 210 and outputs the calculated staying time information to the smart phone 300 or the like.

3. Relational expression between a zone staying time and body weight fluctuation

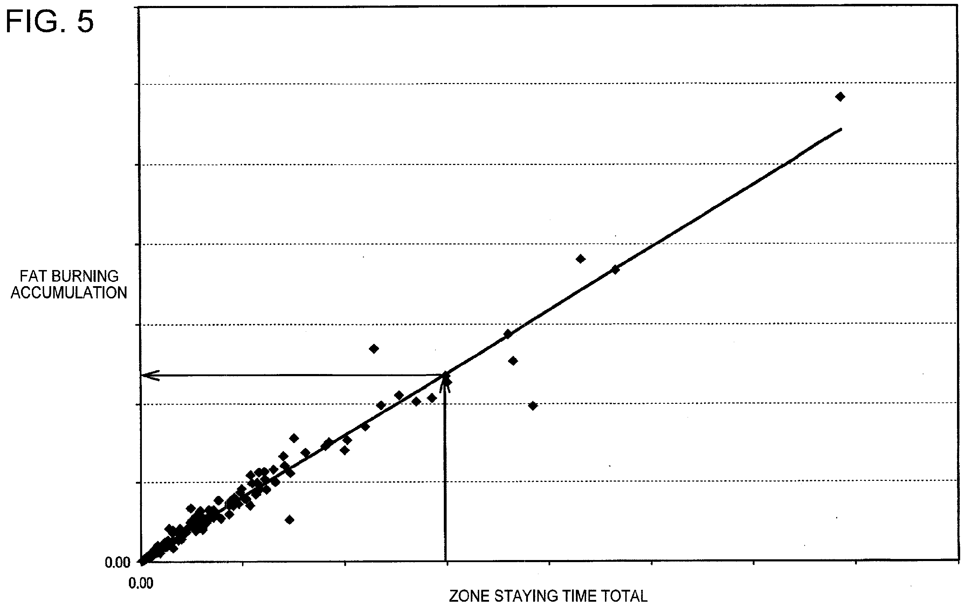

As explained above, in this embodiment, advice information is created on the basis of a zone staying time, which is time in which a value of a pulse rate or the like is present in a fat burning zone. Specifically, a relational expression for associating body weight fluctuation or body fat amount fluctuation and the zone staying time is calculated. Advice information is created using the relational expression. In the following explanation, the relational expression is derived using Fig. 5 and the like and appropriateness of the relational expression is explained.

Fig. 5 is a diagram showing a relationbetween a cumulative value of a zone staying time and a cumulative value of a fat burning amount. Specifically, the cumulative value of the zone staying time is plotted on the abscissa and the cumulative value of the fat burning amount is plotted on the ordinate. Measured values obtained from user data are plotted in the figure.

As it is evident from Fig. 5, it is seen that the cumulative value of the zone staying time and the cumulative value of the fat burning amount indicate a high correlation. As shown in Fig. 5, plotted points are distributed around a regression straight line. A calculated correlation coefficient is a value close to 1.

That is, from an analysis of Fig. 5 performed using the measured values, it is seen that there is a high correlation between the zone staying time and the fat burning amount. In Fig. 5, since correction processing by age, sex, body weight, and the like of the user is not performed, dispersion is caused by the factors. However, the correlation is sufficiently high even if the dispersion factors are included. As a result, for example, a prediction "If you perform exercise for x hours to set a pulse rate to a value within the fat burning zone, maximum y kg of body fat can be reduced" can be performed. In the prediction, it is unnecessary to take into account the age, the sex, the body weight, and the like. As an example, a prediction that, if exercise for keeping a pulse rate within the zone as indicated by an arrow in Fig. 5 is performed 500 hours (=30000 minutes), maximum 24 kg of body fat can be reduced can be performed for users of various ages, sexes, and body weights.

However, values on the ordinate in Fig. 5 are based on the assumption that an entire consumed calorie is used for fat burning, that is, the assumption that a burning amount of fat and an actual decrease amount of body fat are equal. However, actually, it is known that energy used for fat burning is a part of the entire consumed calorie and, when an intake calorie by meals and the like is taken into account, it is highly likely that the fat burning amount is not equal to the decrease amount of the body fat. That is, the prediction is correctly described as follows: if you perform exercise for x hours to set a pulse rate to a value within the fat burning zone, "ideally" , maximum y kg of body fat can be reduced.

In this embodiment, the advice information is specifically information concerning a weight loss. Therefore, the advice information needs to be based on a decrease amount of body weight or a body fat amount conforming to the reality rather than an ideal fat burning amount. That is, whereas it is desired to derive a relation between the zone staying time and the decrease amount of the body weight or the body fat amount, the relation is not sufficiently derived only by the analysis shown in Fig. 5.

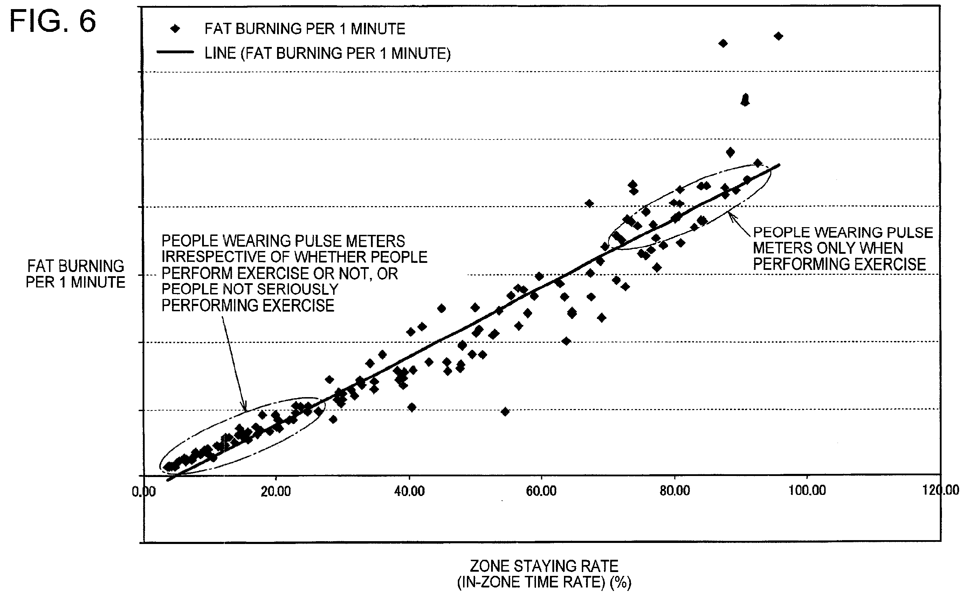

A relation between a rate of the zone staying time per unit time (hereinafter also referred to as zone staying rate) and a fat burning amount per unit time is shown in Fig. 6. The abscissa indicates, for example, percentage indication of the length of the zone staying time in one hour. More specifically, the rate is 100% if a pulse rate is always a value within the fat burning zone for one hour (sixty minutes). The rate is 50% if a pulse rate is within the fat burning zone for thirty minutes and outside the fat burning zone for the remaining thirty minutes.

When the abscissa is plotted as explained above, distributed positions in the graph are different depending on a wearing state of the pulse meter and a state of approach to exercise. For example, a user having a value on the abscissa close to 100% is a user who wears the pulse meter only in a situation in which exercise is performed, that is, a situation in which a pulse rate is higher than a normal value and is a value within the fat burning zone and energetically approaches exercise. On the other hand, a user having a value on the abscissa close to 0% is a user who wears the pulse meter even when the user does not perform exercise or a user who wears the pulse meter only in an exercise state but does not have an enough exercise load, for example, does not energetically approach exercise. That is, it is possible to learn a wearing state of the pulse meter, an exercise state, and the like of each user by viewing the value on the abscissa.

That is, according to the analysis performed using Fig. 6, it is seen that not only the cumulative value of the zone staying time in Fig. 5 but also the zone staying rate has a high correlation with the fat burning amount. In other words, it is seen that the zone staying rate can be used as an index value irrespective of wearing states of the pulse meter and exercise states of users. By using the zone staying rate as the index value, the wearing states of the pulse meter and the exercise states of the users can be estimated as explained above. Therefore, it is also possible to generate advice information corresponding to the states.

However, in Fig. 6, the ordinate also indicates the fat burning amount. Therefore, unless a relation with an actual body fat decrease amount is further examined as in Fig. 5, it cannot be concluded that a decrease amount of body fat can be determined on the basis of the zone staying rate.

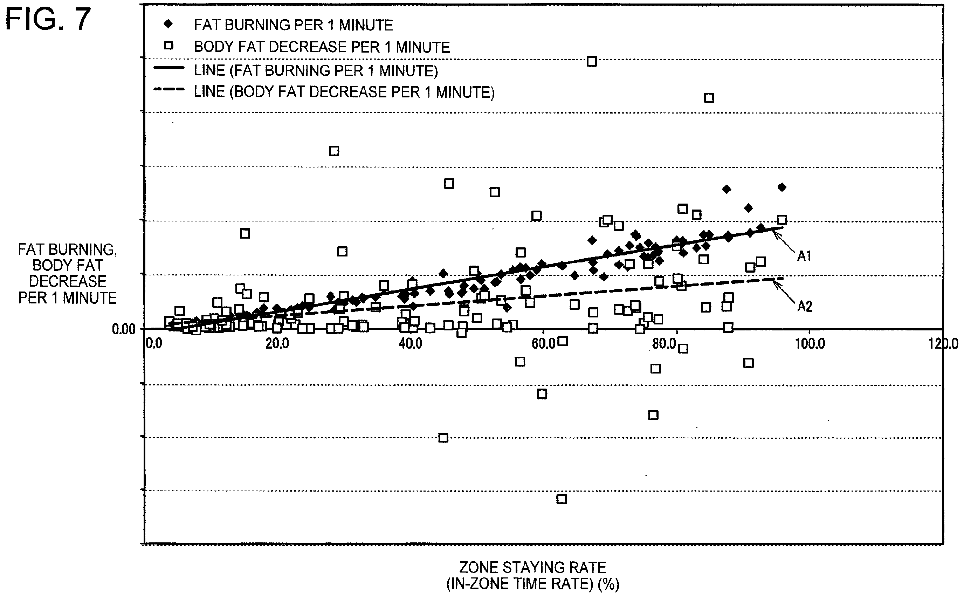

A relation between a zone staying rate and a fat burning amount per unit time and a relation between the zone staying rate and a decrease amount of body fat per unit time are shown in Fig. 7. The zone staying rate and the fat burning amount per unit time are the same as those shown in Fig. 6. It is seen that the zone staying rate and the fat burning amount per unit time have a high correlation.

Concerning the zone staying rate and the decrease amount of body fat per unit time, dispersion is extremely large because an intake calorie is not taken into account. A correlation coefficient is a small value. However, a statistically calculated critical rate is 0.3%. Although the critical rate is low, the correlation between the zone staying rate and the decrease amount of body fat cannot be abandoned. That is, an analysis result is obtained that indicates that, when only the relation between the zone staying rate and the decrease amount of body fat per unit time is taken into account, there is a correlationbetween the zone staying rate and the decrease amount of body fat per unit time that cannot be abandoned.

It is also possible to perform an analysis using both of a regression line (A1) calculated from the zone staying rate and a distribution of the fat burning amount per unit time and a regression line (A2) calculated from the zone staying rate and a distribution of the decrease amount of body fat per unit time. As explained above, since the zone staying rate and the fat burning amount have a high correlation, reliability concerning the regression line A1 is high. It is seen that, when the regression line A2 is calculated from measured values, a tilt of the regression line A2 is approximately a half of a tilt of the regression line A1. In general, a theory that approximately a half of a consumed calorie is used for fat burning is widely known. The fat burning amount, which is one element of the ordinate in Fig. 7, is a value in the case in which it is assumed that the entire calorie is used for the fat burning as explained above. Therefore, if the consumed calorie used for the fat burning is approximately a half of the entire calorie, fat actually burned, that is, the decrease amount of body fat should be a half of the fat burning amount shown in Fig. 6. That is, when it is taken into account that A 1 is a regression line of an ideal fat burning amount (corresponding to the entire consumed calorie), the decrease amount of body fat is theoretically a half of A1, and a tilt of the regression line A2 calculated from a measured decrease amount of body fat is a half of the tilt of A1, it is possible to derive a conclusion that A2 matches a theory as a relational expression representing the decrease amount of body fat.

That is, according to the analysis performed using Fig. 7, it is possible to obtain a result that the regression line A2 is appropriate as a regression line representing a relation between the zone staying rate and the decrease amount of body fat. Note that, when it is taken into account that the high correlation is obtained in both of Fig. 5 and Fig. 6, if a regression line is calculated on the basis of the zone staying time and the decrease amount of body fat, it is possible to consider that the regression line is an appropriate relational expression that determines the relation between the zone staying time and the decrease amount of body fat.

Consequently, when the decrease amount (in a broad sense, a fluctuation amount) of body fat is represented as W, the zone staying time is represented as T_zone, and a coefficient determined by a regression line (a value determined by a tilt of the regression line) is represented as Kfat, a relation among the decrease amount, the zone staying time, and the coefficient is determined by the following Expression (1).

$$\mathrm{T\_zone \times Kfat = W} \qquad\qquad (1)$$

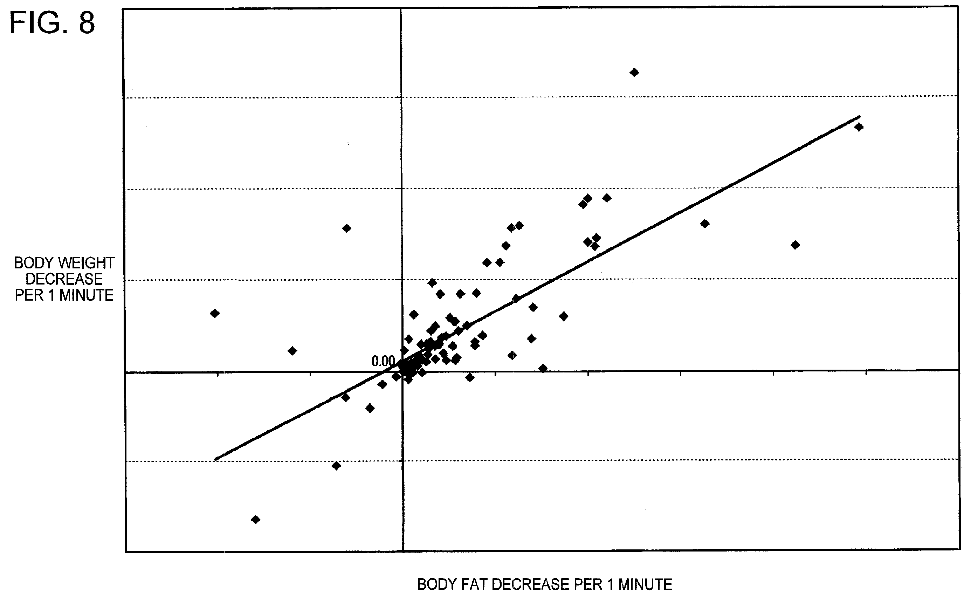

A relation between a decrease amount of body fat and a decrease amount of body weight is shown in Fig. 8. Specifically, the abscissa of Fig. 8 indicates the decrease amount of body fat and the ordinate indicates the decrease amount of body weight. Measured values acquired from the user are plotted in the figure. A tilt of a regression line shown in Fig. 8 is approximately 1. That is, according to an analysis performed using Fig. 8, a conclusion is obtained that the decrease amount of body fat and the decrease amount of body weight may be considered the same.

A value of body weight can be measured by a weight meter widely in use. It is known that an error of the value is not so large. On the other hand, body fat cannot be measured in the first place unless a body meter or the like having a function of a body fat meter is used. Even if a body fat amount (or a body fat rate) can be measured, an error is large compared with the measured value of body weight. That is, information concerning body fat of the user is highly likely to be not input or inaccurately input. That is, although the explanation is limited to body fat in Fig. 5 to Fig. 7 and the above Expression (1), if an analysis result by Fig. 8 is taken into account, the body fat maybe replaced with the body weight. Specifically, it is also possible to treat W as a fluctuation amount of body weight in the above Expression (1) and perform prediction processing.

4. Specific example of processing performed using a relational expression

A method of generating advice information actually using a relational expression indicated as the above Expression (1) is explained. Specifically, processing at advice period start time and processing at advice timing, which is given timing during an advice period, are explained.

The advice period corresponds to a period in which the user (the subject) is provided with a service using the information processing system of this embodiment. For example, a start point of the advice period may be timing when the user applies for the service or may be timing when the user actually starts transmission of pulse wave information or the like. When a service use period is set at the service application time, an end point of the advice period may be timing when the use period elapses from the start point of the advice period. The end point of the advice period may be changed as appropriate according to a request from the user.

The start point of the advice period may be determined from an analysis start point of data rather than being determined from a start of use of the service. For example, it is assumed that a certain user applied for service use in a period of sixth months but was unable to find time for performing exercise in the first three months because of circumstances. In that case, even if the user performs sufficient exercise in the remaining three months, it is likely to be determined that exercise is in insufficient as a whole because of data in the former three months. In that case, a point when three months elapse from the service use start may be reset as a start point of the advice period to cope with the determination.

However, in the same case, rather than resetting the advice period, it may be considered that the advice period starts at the service use start time and two data collection periods (a first month to a third month and a fourth month to a sixth month) in the advice period. That is, the advice period of this embodiment is a concept including both of setting based on the service use start and setting based on the data collection periods. Note that, in the following explanation, the advice period based on the service use start is explained as an example.

4.1 Processing at the advice period start time (target setting processing)

First, target setting processing performed at the advice period start time is explained. Specifically, the target setting processing is processing for receiving an input of body information and target information from the subj ect or the mentor and calculating staying time information required for realizing the target information. Note that the staying time information may be a cumulative value of a zone staying time or may be a zone staying rate. However, in the following explanation, it is assumed that the staying time information is a zone staying time per one day. Either body weight or body fat may be used as explained above. However, in the following explanation, body weight is explained as an example.

As the body information, present body weight $W_{ini}$ (a present value of body weight) of the user is acquired. As the target information, body weight $W_{target}$ (a target value of body weight) targeted by the user and a target period $T_{target}$ (days), which is a period for realizing the body weight, are acquired.

In this case, if T_zone represents a zone staying time per one day, the above Expression(1) can be modified like the following Expression (2). As a result, T_zone is calculated by the following Expression (3). Note that, in the following Expressions (2) and (3), since a decrease amount of body weight is a positive value, in the right side, the target value is subtracted from the present value of body weight.

$$\mathrm{T\_zone} \times T_{target} \times Kfat = W_{ini} - W_{target} \qquad (2)$$

$$T\_zone=(W_{ini}-W_{target})/(T_{target}\times Kfat) \qquad (3)$$

In the right side of the above Expression (3), $W_{ini}$, $W_{target}$, and $T_{target}$ are acquired by the information acquiring unit 110. Kfat is calculated from statistics of Fig. 7 and the like as explained above. That is, by applying the acquired information to the above Expression (3), it is possible to calculate, as T_zone, a zone staying time per one day requested to realize the target body weight in the target period.

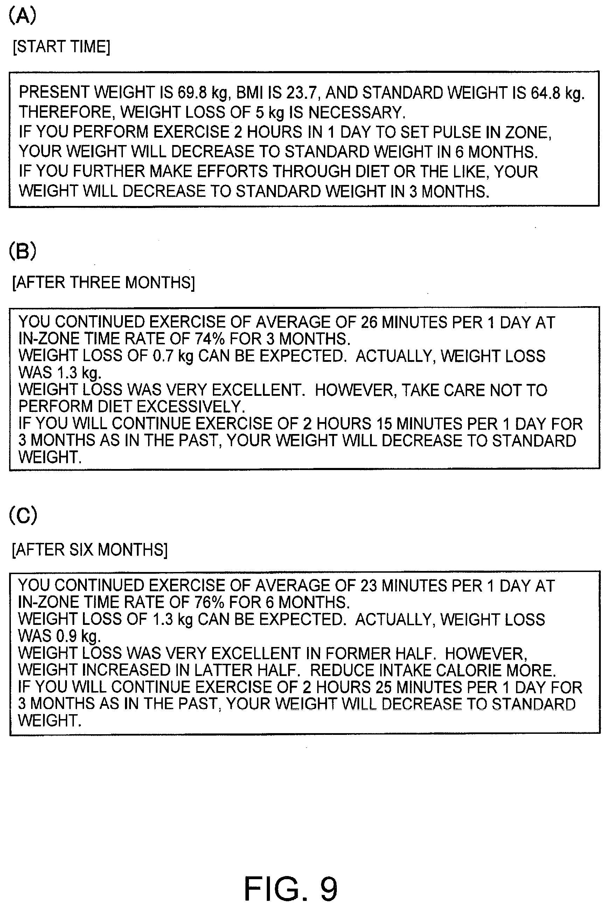

As the advice information at the advice period start time, information "By setting the zone staying time per one day to T_zone, the weight can be reduced to $W_{target}$ after $T_{target}$" only has to be present as in a display screen example shown in Fig. 9(A).

Note that, after T_zone is calculated, when a value of calculated T_zone is unreasonable, resetting of the target information may be instructed to the subject or the like. For example, when T_zone, which is the zone staying time per one day, is a value exceeding twenty-four hours, such exercise is impossible by any means. Even if exercise is not impossible in terms of a numerical value, if the zone staying time in one day is approximately five hours, not many users can afford to consume the time for exercise. The exercise is not considered unrealistic. This is caused by deviation of target body weight from the present body weight or a short target period or both. That is, since sudden body weight fluctuation in a short period is set as a target, a realistically difficult exercise amount is advised.

Therefore, in this embodiment, a series of processing for determining calculated T_zone and, when T_zone is not a reasonable value, instructing a change of target information, and calculating T_zone again with the target information after correction. Consequently, T_zone can be set to a realizable value. Therefore, it is possible to perform exercise conforming to the advice. Note that the determination concerning whether T_zone is reasonable only has to be performed in, for example, comparison processing with a given threshold. When T_zone is equal to or smaller than the threshold, T_zone is determined as reasonable. However, since a reasonable exercise time is different depending on an occupation, a lifestyle, and the like of the user, the threshold is set for each user.

4.2 Processing at advice timing

Processing at advice timing is explained. Specifically, an advice concerning an intake calorie, an advice concerning an exercise amount, an example in which the target setting processing is performed again, and a modified example are explained. Note that the advice timing is timing when a request for advice information is performed. The advice timing may be set in advance to every one month or the like. When the subject or the mentor performs advice request processing according to necessity, the advice timing may be request timing of the advice request processing.

4.2.1 Creation processing for advice information concerning an intake calorie

Information concerning a zone staying time from a start point of the advice period to the advice timing is input as appropriate from the user. For example, if the zone staying time is processed per one day, information such as a zone staying time T_zone1 of the first day and a zone staying time T_zone2 of the second day is input. However, data of pulse wave information (a pulse rate) of an input may be not changed. Processing for performing comparison with a fat burning zone to calculate the zone staying time may be performed in the information acquiring unit 110.

In this case, when a cumulative value of the zone staying time in a data collection period (in a narrow sense, in a period from the start of the advice period to the advice timing) is represented as $\Sigma T\_zone$, the above Expression (1) can be modified as the following Expression (4). As a result, $W_{target}$ is calculated by the following Expression (5). Note that a decrease amount of body weight is a positive value in the following expressions (4) and (5) as in the above Expressions (2) and (3).

$$\Sigma T\_zone \times Kfat = W_{ini} - W_{target} \qquad (4)$$

$$W_{target} = W_{ini} - \Sigma T\_zone \times Kfat \qquad (5)$$

When considered from a measured zone staying time, $W_{target}$ is an estimated value of body weight. The both weight of the user at the advice timing is expected to be this value.

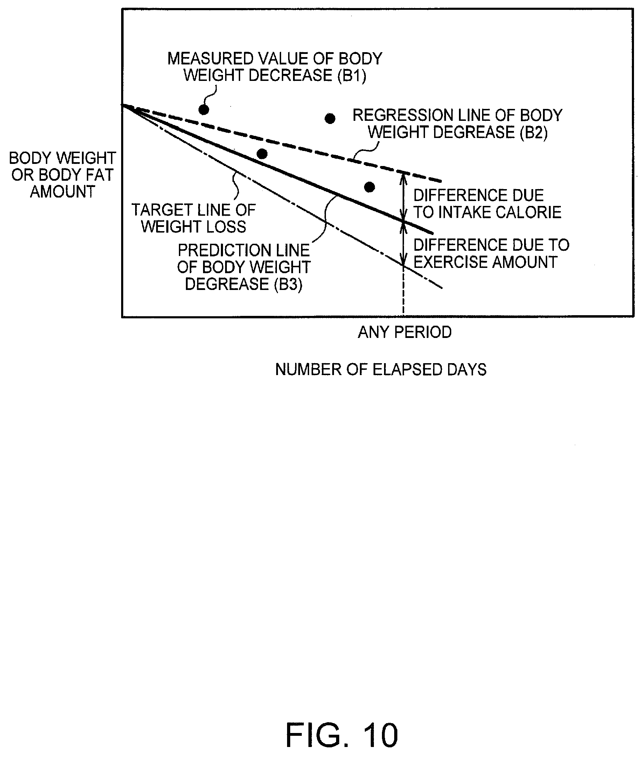

At the advice timing, separately from the estimated value of body weight calculated by the above Expression (5), measured values of body weight input from the user everyday are also acquired as body information. Dispersion of the measured values of body weight is large depending on a day as indicated by B1 of Fig. 10. Therefore, a regression line B2 is calculated and used as information representing a measured value of a body weight decrease.

At this point, the estimated value calculated by the above Expression (5) and the measured value calculated from the regression line as shown in Fig. 10 should ideally coincide with each other. If the estimated value and the measured value do not coincide with each other, some cause is considered to be present. Therefore, examination is performed.

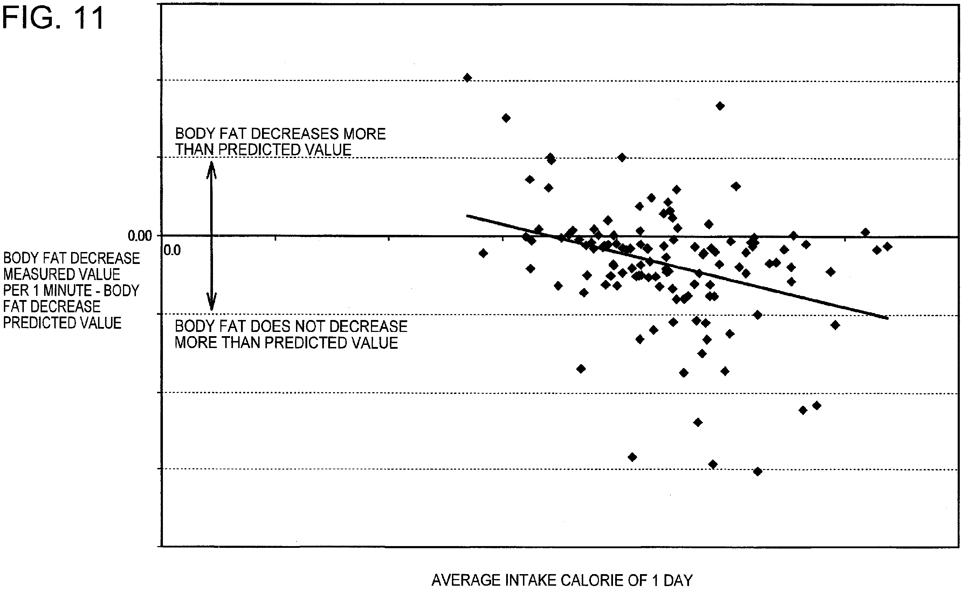

A relation between an average intake calorie in one day and a difference between a measured value and an estimated value of abody fat decrease is shown in Fig. 11. The abscissa indicates the average intake calorie and the ordinate indicates a difference value between the measured value and the estimated value of the body fat decrease. As explained above, the ordinate can be considered the same as a difference value between a measured value and an estimated value of a body weight decrease.

As shown in Fig. 11, although dispersion of values is large, a tendency is found that a value of the ordinate is a smaller value (a negative value) as the average intake calorie is larger and a value of the ordinate is larger value (a very small negative value to a positive value) as the average intake calorie is smaller. The negative value of the ordinate corresponds to a case in which the measured value of the body fat decrease is smaller than a predicted value, that is, a body fat decrease as predicted is not found. That is, according to an analysis performed using Fig. 11, it is possible to derive a conclusion that the measured value of the body weight decrease smaller than the predicted value corresponds to a larger intake calorie and the measured value of the body weight decrease larger than the predicted value corresponds to a small intake calorie.

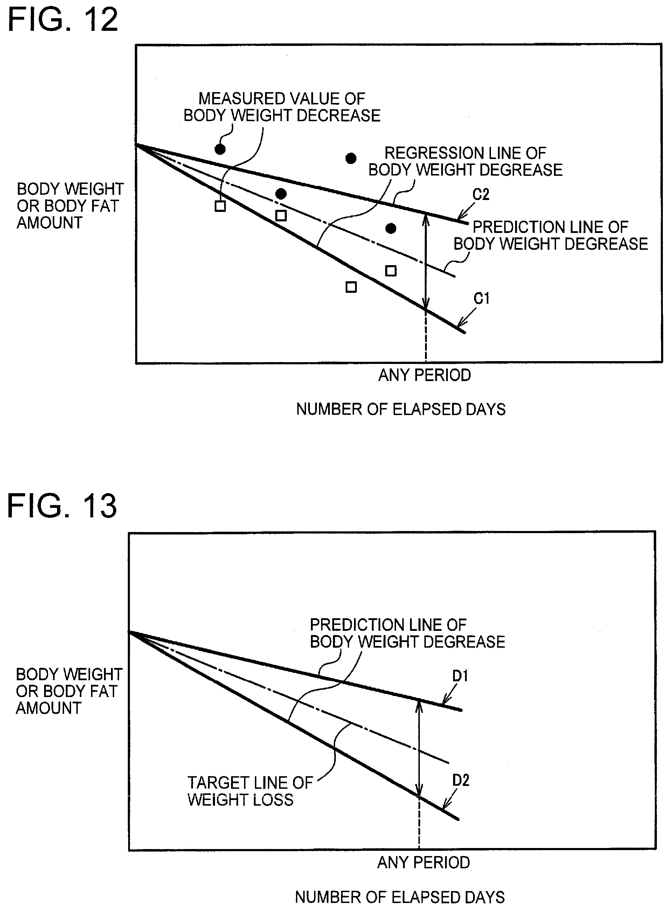

That is, it is possible to compare a prediction line representing body weight fluctuation determined by the estimated value calculated from the above Expression (5) and a regression line of a body weight decrease determined from a plot of measured values of body weight and estimate an amount of an intake calorie according to which of the prediction line and the regression line is above the other. Specifically, if the regression line is below the prediction line as indicated by C1 in Fig. 12, it is determined that the intake calorie is small. If the regression line is above the prediction line as indicated by C2 in Fig. 12, it is determined that the intake calorie is large. In the example shown in Fig. 10, the regression line B2 is above the prediction line B3 of the body weight decrease determined from Expression (5). Therefore, it canbe determined that an intake calorie amount of a target user is large.

Examples of advice information at the advice timing are shown in Fig. 9(B) and Fig. 9(C). A zone staying rate of 74%, a zone staying time of 26 minutes/day, and a collection period of three months shown in Fig. 9(B) indicate measured values in a period to the advice timing. 0.7 kg is an estimated value calculated from the above Expression (5) of a body weight decrease expected when a zone staying time of 26 minutes/day is continued for three months. 1.3 kg is a value calculated using a regression line of a measured value of body weight input from the user. An example shown in Fig. 9(B) corresponds to a case in which a regression line is below a prediction line. A reduction in an intake calorie is evaluated as "very excellent". An expression for restraining an excessive diet is included in the advice information.

In an example shown in Fig. 9(C), numerical values are the same as the numerical values in Fig. 9(B). However, a measured value (0.9 kg) calculated from a regression line is small with respect to a predicted value (1.3 kg) of a body weight decrease. This corresponds to an excess intake calorie. Therefore, an expression for restraining an intake calorie is included in the advice information. Note that, in Fig. 9(B), an example after three months from the start of the advice period is assumed and, in Fig. 9(C), an example after six months is assumed. Therefore, the advice information shown in Fig. 9 (C) includes an expression ("very excellent in the former half") that takes into account a result at the advice timing in Fig. 9(B).

4.2.2. Creation processing for advice information concerning an exercise amount

Advice information concerning an exercise amount may be created at the advice timing. An estimated value is calculated from a measured value of the zone staying time using the above Expression (5) in the same manner as explained above. A difference from the advice information concerning the intake calorie is that a comparison target with a prediction line is not a regression line but a target line set by the target setting processing explained above. In the target setting processing, a target body weight at the time of elapse of a target period is calculated. Therefore, by using a value of the target body weight and the present value, it is possible to set the target line as indicated by B3 in Fig. 10.

In this advice information generation processing, specifically, if the target line is below the prediction line as indicated by D1 in Fig. 13, it is determined that the exercise amount is not enough for expecting a body weight decrease equivalent to the target setting and is insufficient. On the other hand, the target line is above the prediction line as indicated by D2 in Fig. 13, it is determined that the exercise amount is enough for expecting a body weight decrease equal to or larger than the target setting and is large.

However, a target exercise amount corresponds to a zone staying time calculated by the target setting. An actual exercise amount corresponds to a measured value of the zone staying time. Therefore, rather than respectively calculating the prediction line and the target line, the exercise amount maybe determined from comparison processing of the target value and the measured value of the zone staying time. It is also likely that the data collecting period to the advice timing and the target period do not coincide with each other. Therefore, it is inappropriate to compare a cumulative value of the zone staying time. However, comparison processing of a zone staying time per unit time may be performed.

### 4.2.3 Resetting processing for a target

As explained above, it is conceivable that the target value of the body weight fluctuation set at the advice period start time and the measured value of the body weight fluctuation at the advice timing are different values because of at least one factor of the intake calorie and the exercise amount. In that case, values of the zone staying time and the like set at the advice period start time are not appropriate in realizing the target value of body weight in the target period.

For example, it is assumed that the processing is performed with the target period set to sixth months and with timing after the elapse of three month set as the advice timing. If a target after sixth months is used as it is, when a body weight decrease is inprogress at speed higher than a target because, for example, an intake calorie is reduced for three months or an exercise amount is increased, the target can be attained even if a weight loss pace is slowed by relatively increasing the intake calorie in the latter three months or reducing the exercise amount. Conversely, if the weight loss pace is slow in the former half, even if the zone staying time set in the beginning is kept, the target weight cannot be realized when sixth months elapse.

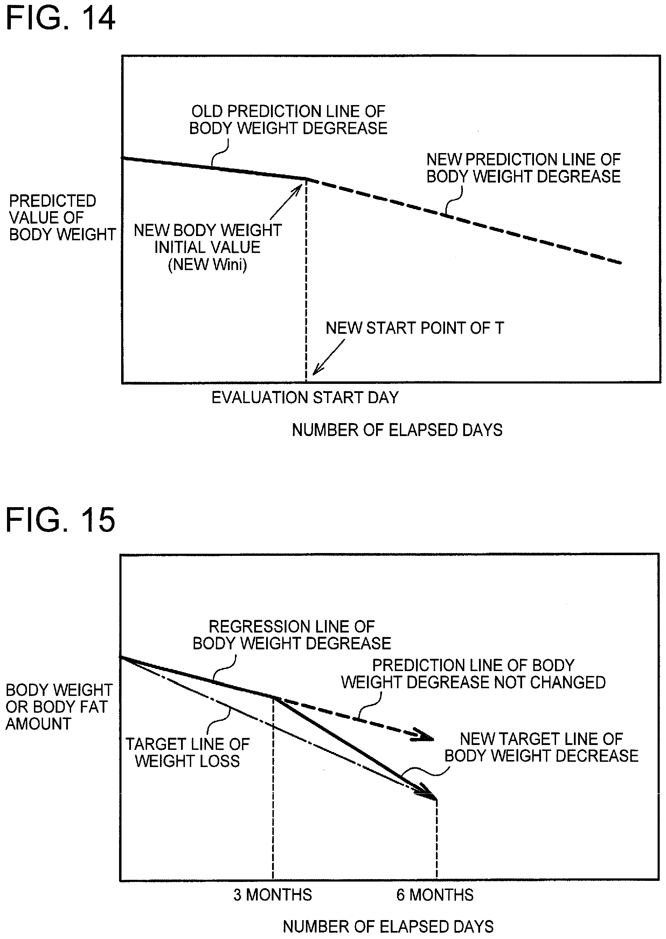

That is, the target setting processing performed at the advice period start time is not limited to the target setting processing performed at the timing and may be performed at the advice timing. Since specific processing content is the same as the processing content explained above, detailed explanation of the specific processing content is omitted. However, in the above Expression (1), $W_{ini}$ is not the value at the advice period start time. A measured value at the advice timing is used as shown in Fig. 14. The target period T needs to be changed to a period starting from the advice timing.

The processing explained above is shown in Fig. 15. Fig. 15 corresponds to an example in which, since a weight loss pace is slow halfway, a value larger than the initial zone staying time is set after the advice timing and the target is changed to a stricter target.

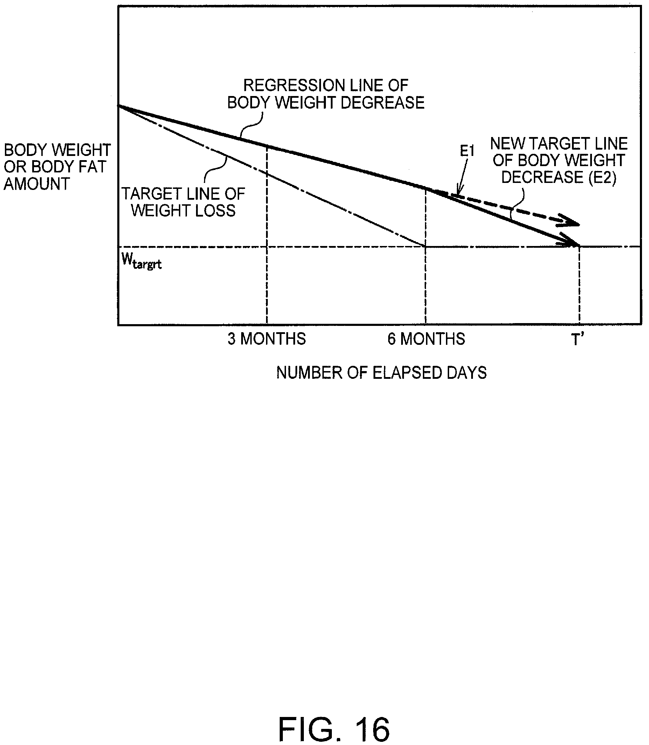

As shown in Fig. 16, the generation processing for the advice information performed using the above Expression (5) and the like and the resetting processing for a target may be performed at the end of the advice period. In an example shown in Fig. 16, even when six months of the advice period elapses, the initial target cannot be attained. It is assumed that the service provision according to this embodiment ends unless an application for extension or the like of the advice period is not performed. However, it is useful to generate advice information at the advice period end time in terms of giving a guideline for attaining an unattained target thereafter.

For example, in an example shown in Fig. 16, when equivalent exercise or the like is continued by extending, as indicated by E1, a regression line calculated from a measured value of a body weight decrease in the advice period, a period or the like required for realizing the original target body weight $W_{target}$ is generated as the advice information. Alternatively, appropriate target timing T' may be set and a new target line E2 for realizing the original target body weight $W_{target}$ by the target timing T' may be set. As explained using the above Expression (3), if a target line is set, the zone staying time T_zone requested to realize the target line can be calculated. It is possible to present the calculated T_zone as advice information.

### 4.2.4 Modified example

A modified example of the advice information generation processing is explained. In the processing explained above, it is assumed that the advice information generation processing is performed at the predetermined advice timing such as once a month. The advice timing is set by the subject or the mentor.

However, it is assumed that an interval of the advice timing is large to a certain degree. This is because, even if advice information is frequently generated, a change in a situation is little and the advice information is considered not useful. Therefore, even if fluctuation in a state of an intake calorie, an exercise state, and the like occurs between advice timing and advice timing, the fluctuation is not presented to the subject and the like as advice information until the next advice timing.

For example, even if the subject intentionally changes an intake calorie amount and an exercise amount, whether the change is effective is presented at the next advice timing. Therefore, it is also likely that time is required until the presentation depending on setting of the advice timing. Alternatively, since it is undesirable that an exercise state or the like changes without being intended by the user, whereas a state change shouldbe quickly presented to the user, similarly, it is likely that time is required until the presentation. For example, although the user himself/herself thinks that the user is continuing exercise of the same degree, when it is determined that a situation with a sufficient exercise amount changes to a situation with an excessively small exercise amount, it is likely that time is required until the user becomes aware of fluctuation to a situation undesirable for health maintenance. Concerning fluctuation from the situation with the excessively small exercise amount to the situation with the sufficient exercise amount, although the fluctuation itself is desirable concerning a body weight decrease, the unawareness of the fluctuation by the user indicates a risk of performing excessive exercise unconsciously and cannot be overlooked.

Therefore, as a modified example, the information processing system may perform processing for executing the comparison processing of the prediction line and the regression line or the prediction line and the target line in the background and, when a change occurs in a situation of an intake calorie or the like, even if timing of the change is not the advice timing, actively distributing the advice information to the subject and the mentor.

Specifically, the information processing system performs the processing concerning the intake calorie and the processing concerning the exercise amount at given processing timing different from the advice timing. The processing timing may be set, for example, at a given interval shorter than the interval of the advice timing. Note that, although the advice information is not always output at the timing, the processing timing is included in a concept of the advice timing in a broad sense in terms of having possibility of an output. That is, the advice timing of this embodiment includes advice timing in a narrow sense (the advice timing explained above) when the output of the advice information is decided and advice timing in a broad sense (processing timing) when the advice information is output only when it is determined that a change of a situation occurs. Processing content at the processing timing is explained below.

For example, the information processing system 100 may present information when a positional relation between a prediction line and a regression line at the latest processing timing is different from a positional relation at the immediately preceding processing timing. Specifically, it is assumed that, at first processing timing, it is determined that a prediction line is above a regression line and an intake calorie is large and, at the next second advice timing, it is determined that the regression line is below the prediction line and the intake calorie is small. In this case, a calorie in an excessive intake state is changed to an excessively small intake state in a period from the first processing timing to second processing timing. Information concerning the change is information useful for the subject and the mentor. Therefore, the information processing system 100 outputs advice information to the effect that the intake calorie changes to the subject and the like. As an example, it is conceivable to generate advice information "The weight decreases more than fat burning. The diet is likely to be hard".

On the other hand, when a state in which the prediction line is lower than the regression line changes to a state in which the prediction line is above the regression line, it is seen that the intake calorie changes to increase. Therefore, for example, advice information "The weight does not decrease as much as fat burning. Try to reduce an intake calorie" only has to be generated. Consequently, it is possible to present, in a timely manner, information such as overeating and an excessive diet to the subject and the mentor.

Note that, since a slight difference can be considered an error range, necessary advice information does not have to be presented when a relation between the straight lines reverses. For example, the advice information may be presented when the relation reverses and an amount of change exceeds a threshold.

Advice information concerning an exercise amount can be considered the same as the intake calorie. For example, it is assumed that, at the first processing timing, it is determined that the prediction line is above the target line and the exercise amount is insufficient and, at the next second advice timing, it is determined that the prediction line is below the target line and the exercise amount is sufficient. In this case, a small exercise amount is changed to a large exercise amount in the period from the first processing timing to the second processing timing. Therefore, it is conceivable to generate advice information "Exercise is likely to be hard. Continue the exercise without strain".

On the other hand, when a state in which the prediction line is below the target line changes to a state in which the prediction line is above the target line, it is seen that the exercise amount changes to decrease. Therefore, for example, advice information "To attain the target, set an exercise time longer" only has to be generated. Consequently, it is possible to present, in a timely manner, information such as a lack of exercise and excessive exercise to the subject and the mentor.

5. Specific example of this embodiment

In this embodiment explained above, the information processing system 100 includes, as shown in Fig. 2, the information acquiring unit 110 that performs acquisition processing for information, the processing unit 120 that performs processing on the basis of the information acquired by the information acquiring unit 110, and the output processing unit 130 that performs output processing for information created by the processing unit 120. The information acquiring unit 110 performs acquisition processing for at least two kinds of information among staying time information representing time in which a value represented by pulse wave information of the user is a value within a given fat burning zone, body information of the user, and target information concerning a weight loss of the user. The processing unit 120 performs processing for creating advice information concerning a weight loss on the basis of a relational expression representing a relation between fluctuation in body weight or a body fat amount of the user and the staying time information and the at least two kinds of information acquired by the information acquiring unit 110. The output processing unit 130 performs output processing for the advice information created by the processing unit 120.

The value represented by the pulse wave information may be a pulse rate in a narrow sense but is not limited to the pulse rate. For example, the value represented by the pulse wave information may be information such as a frequency of an AC component of a pulse wave signal. The fat burning zone is a range determined on the basis of a value represented by a standard pulse wave signal and represents a range suitable for fat burning. For example, if the value represented by the pulse wave signal is the pulse rate, the fat burning zone is a numerical value range of a pulse rate suitable for fat burning. Note that, as a method for calculating the pulse rate (a heart rate) suitable for fat burning, various methods such as a Karvonen Formula are known. Therefore, detailed explanation of the method is omitted. As a determination method for the fat burning zone in this embodiment, various methods can be optionally applied. The determination method is not limited to a specific method. The staying time information only has to be information representing time in which a value represented by a pulse wave signal is a value within the fat burning zone. The staying time information may be a cumulative value of the zone staying time or may be a zone staying time in a unit period (e.g. , one day). The staying time information may be a zone staying rate, which is a rate of the zone staying time in a unit time (e.g. , one hour). The body information is information such as body weight and a body fat amount of the user and may include information such as sex, height, and age in a broad sense. The target information is information serving as a target of a weight loss and is a target value of body weight, a target value of a body weight decrease, a target period until the body weight decrease is realized.

Consequently, after acquiring at least two of the staying time information, the body information, and the target information, it is possible to generate advice information according to processing performed using a relational expression between the staying time information and body weight fluctuation (or fluctuation in body fat). Therefore, information to be generated is generated from a viewpoint of staying time information. For example, a zone staying time requested when target body weight is realized within a target period is shown. Therefore, when it is attempted to realize a weight loss through exercise, it is possible to suppress, for example, excessive exercise in which a pulse rate exceeds an upper limit of the fat burning zone and occurrence of injury because of the excessive exercise. By appropriately setting the fat burning zone, it is possible to execute exercise with a load with which fat burning can be efficiently performed. Further, a decrease in motivation due to setting of an excessive exercise target or the like can be suppressed. Therefore, it is possible to perform a continuous service use.

When a value corresponding to an integrated value of staying time information in a given period is represented as T_zone, a fluctuation amount of body weight or a body fat amount of the user in a given period is represented as W, and a fat burning coefficient is represented as Kfat, a relational expression may be T_zone×Kfat=W as indicated by the above Expression (1).

The value corresponding to the integrated value of the staying time information is, in the above Expression (2), a product of a zone staying time per one day (T_zone in the above Expression (2)) and the period T. In the above Expression (4), the value is a value obtained by accumulating, in a period, a measured zone staying time in a unit period (T_zone in the above Expression (4)). However, the integrated value of the staying time information may be calculated by other methods. The fat burning coefficient is a coefficient for associating T_zone and W. It is conceivable that the fat burning coefficient is determined from, for example, a tilt of A2 in Fig. 7 or a straight line similar to A2.

Consequently, it is possible to perform generation processing for advice information in which the above Expression (1) is used. It is possible to appropriately associate the staying time information with a fluctuation amount of body weight or the like.

The information acquiring unit 110 may perform acquisition processing for staying time information and body information. The processing unit 120 may perform processing for creating advice information concerning an intake calorie of the user on the basis of the staying time information, the body information, and the relational expression.

Specifically, the information acquiring unit 110 may perform acquisition processing for staying time information up to given advice timing within an advice period and perform acquisition processing for acquiring, as body information, a measured value of body weight or a body fat amount of the user up to the advice timing. The processing unit 120 may perform processing for calculating an estimated value of the body weight or the body fat amount at the advice timing corresponding to the staying time information on the basis of the staying time information and the relational expression and creating advice information concerning an intake calorie on the basis of comparison processing of the calculated estimated value and the measured value.

The estimated value of the body weight or the body fat amount may be a value corresponding to an absolute value of the body weight or the like or may be a fluctuation value with respect to a given reference value (e.g., a value at advice period start time). Concerning the measured value, either an absolute value or a fluctuation value may be used. A correspondence relation between the estimated value and the measured value only has to hold.

Consequently, it is possible to generate advice information concerning an intake calorie as the advice information concerning the weight loss from the staying time information, the body information, and the relational expression. Even if information concerning meals is not input, it is possible to estimate from the information whether a meal amount is "large" or "small". By comparing meal information input by the user and an intake calorie determined by the method of this embodiment, it is also possible to generate advice information concerning, for example, accuracy of the input. The estimated value is equivalent to the prediction line explained above, the measured value is equivalent to the regression line explained above, and the comparison processing is equivalent to the processing explained above with reference to Fig. 12.

The information acquiring unit 110 may perform acquisition processing for staying time information and target information. The processing unit 120 may perform processing for creating advice information concerning an exercise amount of the user on the basis of the staying time information, the target information, and the relational expression.

Specifically, the information acquiring unit 110 may perform acquisition processing for staying time information up to given advice timing within an advice period and perform acquisition processing for acquiring, as target information, a target value of body weight or a body fat amount of the user at advice timing set before the advice timing. The processing unit 120 may perform processing for calculating an estimated value of the body weight or the body fat amount corresponding to the staying time information on the basis of the staying time information and the relational expression and creating advice information concerning an exercise amount on the basis of comparison processing of the calculated estimated value and the target value.

Consequently, it is possible to generate advice information concerning an exercise amount as the advice information concerning the weight loss from the staying time information, the target information, and the relational expression. If the exercise amount is large, the exercise amount leads to a body weight decrease. Therefore, basically, an evaluation is high when the exercise amount is large. However, since excessive exercise causes injury, advice information for urging to properly hold down exercise only has to be generated. The estimated value is equivalent to the prediction line explained above, the target value is equivalent to the target line explained above, and the comparison processing is equivalent to the processing explained above with reference to Fig. 13.

The information acquiring unit 110 may perform acquisition processing for acquiring, as body information, a present value of body weight or a body fat amount of the user at advice timing and perform acquisition processing for acquiring, as target information, a target value of the body weight or the body fat amount of the user and a target period representing a period until the target value is attained. The processing unit 120 may perform processing for calculating, on the basis of the present value, the target value, the target period, and the relational expression, staying time information necessary for attaining the target value within the target period.

Consequently, as explained above with reference to Fig. 14 to Fig. 16, it is possible to perform the resetting processing for a target even at timing halfway in or at the end of the advice period. Therefore, it is possible to generate advice information further reflecting interim progress.

The information acquiring unit 110 may perform acquisition processing for body information and target information. The processing unit 120 may perform processing for calculating, on the basis of the body information, the target information, and the relational expression, staying time information necessary for attaining a target represented by the target information.

Specifically, the information acquiring unit 110 may perform acquisition processing for acquiring, as body information, a present value of body weight or a body fat amount of the user and perform acquisition processing for acquiring, as target information, a target value of the body weight or the body fat amount of the user and a target period representing a period until the target value is attained. The processing unit 120 may perform processing for calculating, on the basis of the present value, the target value, the target period, and the relational expression, staying time information necessary for attaining the target value within the target period.

Consequently, it is possible to perform the target setting processing. Specifically, as shown in Fig. 9(A), it is possible to present the staying time information requested for attaining a target to the user. Specifically, for example, the value of T_zone calculated using the above Formula (3) only has to be presented. By presenting the advice information generated by using the staying time information, it is possible to suppress exercise with an excessive load and efficiently perform fat burning by exercise as explained above.

When a staying time represented by the calculated staying time information is larger than a given threshold, the processing unit 120 may perform processing for generating correction instruction information for instructing correction of at least one of the target value or the target period. When the information acquiring unit 110 performs acquisition processing for at least one of the target value and the target period corrected on the basis of the correction instruction information, the processing unit 120 may perform processing for calculating again, on the basis of at least one of the target value and the target period after the correction, the staying time information necessary for attaining the target value within the target period

Consequently, it is possible to perform reasonable target setting. When excessively strict target setting is performed, in some case, a zone staying time to be calculated is long and it is realistically difficult to perform exercise for a long time that satisfies the zone staying time. If an exercise load may be high as in the conventional method, it is possible to reduce an exercise time. However, in this embodiment, exercise with an excessive load is suppressed from a viewpoint of injury prevention and the like. Therefore, as a result, exercise for a long time is requested for strict target setting. It is difficult to realize the exercise. Therefore, to prevent an unreasonable exercise time from being instructed, it is desirable to perform an instruction for correcting a target to be loose. Note that correction instruction information is, for example, display control information for displaying an input screen for target information again.

The information acquiring unit 110 may perform acquisition processing for body information and target information at start timing of the advice period. The processing unit 120 may perform processing for calculating, on the basis of the body information, the target information, and the relational expression, staying time information necessary for attaining a target represented by the target information.

Consequently, it is possible to perform the target setting processing at the start of the advice period. Note that, as explained above, the target setting processing may be performed again at other timing during the advice period.

Note that a part or a most of the processing of the information processing system 100 and the like of this embodiment may be realized by a program. In this case, a processor such as a CPU executes the program, whereby the inf ormationprocessing system 100 and the like of this embodiment are realized. Specifically, the program stored in a non-transitory information storage medium is read out and the processor such as the CPU executes the read-out program. The information storage medium (a computer-readable medium) stores programs, data, and the like. Functions of the information storage medium can be realized by an optical disk (a DVD, a CD, etc.), a HDD (a hard disk drive), a memory (a card type memory, a ROM, etc.), or the like. The processor such as the CPU performs the various kinds of processing of this embodiment on the basis of the programs (the data) stored in the information storage medium. That is, in the information storage medium, programs for causing a computer (an apparatus including an operation unit, a processing unit, a storing unit, and an output unit) to function as the units of this embodiment (programs for causing the computer to execute the processing of the units) are stored.

Note that, whereas this embodiment is explained in detail above, those skilled in the art could easily understood that many modifications not substantially departing from the new matters and the effects of the present invention are possible. Therefore, all of such modifications are deemed to be included in the scope of the present invention. For example, terms described together with broader or synonymous different terms at least once in the specif ication or the drawings can be replaced with the different terms in any place in the specification or the drawings. The configurations and the operations of the information processing system are not limited to those explained in this embodiment. Various modified implementations of the configurations and the operations are possible.

Reference Signs List

| | |
|---|---|
| 10 | Platform |
| 11 | Operation management service |
| 12 | Pulse machine connection interface |
| 13 | Platform main body |
| 14 | Common database |
| 15 | Advice engine |
| 16 | Prediction engine |
| 100 | Information processing system |
| 110 | Information acquiring unit |
| 120 | Processing unit |
| 130 | Output processing unit |
| 200 | Pulse meter |
| 210 | Pulse wave sensor |
| 300 | Smart phone |
| 310 | Receiving unit |
| 320 | Transmitting unit |
| 330 | Operation unit |
| 340 | Display unit |
| 350 | Information acquiring unit |
| 351 | Receiving unit |
| 353 | Operation unit |
| 360 | Processing unit |
| 370 | Display control unit |
| 380 | Display unit |
| 400 | Server system |
| 410 | Receiving unit |
| 420 | Processing unit |
| 430 | Transmitting unit |

**Claims**

1. An information processing system comprising:

an information acquiring unit that performs acquisition processing for information;
a processing unit that performs processing on the basis of the information acquired by the information acquiring unit; and
an output processing unit that performs output processing for the information created by the processing unit, wherein
the information acquiring unit performs the acquisition processing for at least two kinds of information among staying time information representing time in which a value represented by pulse wave information of a user is a value within a given fat burning zone, body information of the user, and target information concerning a weight loss of the user,
the processing unit performs processing for creating advice information concerning the weight loss of the user on the basis of a relational expression representing a relation between fluctuation in body weight or a body fat amount of the user and the staying time information and the at least two kinds of information acquired by the information acquiring unit, and
the output processing unit performs the output processing for the advice information created by the processing unit.

2. The information processing system according to claim 1, wherein, when a value corresponding to an integrated value of the staying time information in a given period is represented as T_zone, a fluctuation amount of the body weight or the body fat amount of the user in the given period is represented as W, and a fat burning coefficient is represented as Kfat, the relational expression is $T\_zone \times Kfat = W$.

3. The information processing system according to claim 1 or 2, wherein
the information acquiring unit performs the acquisition processing for the staying time information and the body information, and
the processing unit performs processing for creating the advice information concerning an intake calorie of the user on the basis of the staying time information, the body information, and the relational expression.

4. The information processing system according to claim 3, wherein
the information acquiring unit performs the acquisition processing for the staying time information up to given advice timing within an advice period and performs the acquisition processing for acquiring, as the body information, a measured value of the body weight or the body fact amount of the user up to the advice timing, and
the processing unit performs processing for calculating an estimated value of the body weight or the body fat amount at the advice timing corresponding to the staying time information on the basis of the staying time information and the relational expression and creating the advice information concerning the intake calorie on the basis of comparison processing of the calculated estimated value and the measured value.

5. The information processing system according to any one of claims 1 to 4, wherein
the information acquiring unit performs the acquisition processing for the staying time information and the target information, and
the processing unit performs processing for creating the advice information concerning an exercise amount of the user on the basis of the staying time information, the target information, and the relational expression.

6. The information processing system according to claim 5, wherein
the information acquiring unit performs the acquisition processing for the staying time information up to given advice timing within an advice period and performs the acquisition processing for acquiring, as the target information, a target value of the body weight or the body fat amount of the user at the advice timing set before the advice timing, and
the processing unit performs processing for calculating an estimated value of the body weight or the body fat amount corresponding to the staying time information on the basis of the staying time information and the relational expression and creating the advice information concerning the exercise amount on the basis of comparison processing of the calculated estimated value and the target value.

7. The information processing system according to claim 4 or 6, wherein
the information acquiring unit performs the acquisition processing for acquiring, as the body information, a present value of the body weight or the body fat amount of the user at the advice timing and performs the acquisition processing for acquiring, as the target information, a target value of the body weight or the body fact amount of the user and a target period representing a period until the target value is attained, and

the processing unit performs processing for calculating, on the basis of the present value, the target value, the target period, and the relational expression, the staying time information necessary for attaining the target value within the target period.

8. The information processing system according to claim 1 or 2, wherein

the information acquiring unit performs the acquisition processing for the body information and the target information, and

the processing unit performs processing for calculating, on the basis of the body information, the target information, and the relational expression, the staying time information necessary for attaining a target represented by the target information.

9. The information processing system according to claim 8, wherein

the information acquiring unit performs the acquisition processing for acquiring a present value of the body weight or the body fat amount of the user as the body information and performs the acquisitionprocessing for acquiring, as the target information, a target value of the body weight or the body fat amount of the user and a target period representing a period until the target value is attained, and

the processing unit performs processing for calculating, on the basis of the present value, the target value, the target period, and the relational expression, the staying time information necessary for attaining the target value within the target period.

10. The information processing system according to claim 9, wherein

the processing unit performs processing for generating correction instruction information for instructing correction of at least one of the target value and the target period when a staying time represented by the calculated staying time information is larger than a given threshold, and

when the information acquiring unit performs the acquisition processing for at least one of the target value and the target period corrected on the basis of the correction instruction information, the processing unit performs processing for calculating again, on the basis of at least one of the target value and the target period after the correction, the staying time information necessary for attaining the target value within the target period.

11. The information processing system according to any one of claims 8 to 10, wherein

at start timing of an advice period, the information acquiring unit performs the acquisition processing for the body information and the target information, and

the processing unit performs processing for calculating, on the basis of the body information, the target information, and the relational expression, the staying time information necessary for attaining a target represented by the target information.

12. A program for causing a computer to function as:

an information acquiring unit that performs acquisition processing for information;
a processing unit that performs processing on the basis of the information acquired by the information acquiring unit; and
an output processing unit that performs output processing for the information created by the processing unit, wherein
the information acquiring unit performs the acquisition processing for at least two kinds of information among staying time information representing time in which a value represented by pulse wave information of a user is a value within a given fat burning zone, body information of the user, and target information concerning a weight loss of the user,
the processing unit performs processing for creating advice information concerning the weight loss of the user on the basis of a relational expression representing a relation between fluctuation in body weight or a body fat amount of the user and the staying time information and the at least two kinds of information acquired by the information acquiring unit, and
the output processing unit performs the output processing for the advice information created by the processing unit.

13. An information processing method for causing a computer to execute:

acquisition processing for at least two kinds of information among staying time information representing time in which a value represented by pulse wave information of a user is a value within a given fat burning zone, body information of the user, and target information concerning a weight loss of the user;

processing for creating advice information concerning the weight loss of the user on the basis of a relational expression representing a relation between fluctuation in body weight or a body fat amount of the user and the staying time information and the at least two kinds of information acquired by the acquisition processing; and the output processing for the created advice information.

**Amended claims under Art. 19.1 PCT**

1. (Amended) An information processing system comprising:

an information acquiring unit that performs acquisition processing for information;

a processing unit that performs processing on the basis of the information acquired by the information acquiring unit; and

an output processing unit that performs output processing for the information created by the processing unit, wherein

the information acquiring unit performs the acquisition processing for at least two kinds of information among staying time information representing time in which a value represented by pulse wave information of a user is a value within a given fat burning zone, body information of the user, and target information concerning fluctuation in body weight or a body fat amount of the user,

the processing unit performs processing for creating advice information concerning the fluctuation in the body weight or the body fat amount of the user on the basis of a relational expression representing a relation between the fluctuation in the body weight or the body fat amount of the user and the staying time information and the at least two kinds of information acquired by the information acquiring unit, and

the output processing unit performs the output processing for the advice information created by the processing unit.

2. (Amended) The information processing system, wherein the staying time information is a zone staying rate representing a rate of a zone staying time per unit time.

3. (Amended) The information processing system according to claim 1 or 2, wherein, when a value corresponding to an integrated value of the staying time information in a given period is represented as T_zone, a fluctuation amount of the body weight or the body fat amount of the user in the given period is represented as W, and a fat burning coefficient is represented as Kfat, the relational expression is T_zone$\times$Kfat=W.

4. (Amended) The information processing system according to claims 1 to 3, wherein

the information acquiring unit performs the acquisition processing for the staying time information and the body information, and

the processing unit performs processing for creating the advice information concerning an intake calorie of the user on the basis of the staying time information, the body information, and the relational expression.

5. (Amended) The information processing system according to claim 4, wherein

the information acquiring unit performs the acquisition processing for the staying time information up to given advice timing within an advice period and performs the acquisition processing for acquiring, as the body information, a measured value of the body weight or the body fact amount of the user up to the advice timing, and

the processing unit performs processing for calculating an estimated value of the body weight or the body fat amount at the advice timing corresponding to the staying time information on the basis of the staying time information and the relational expression and creating the advice information concerning the intake calorie on the basis of comparison processing of the calculated estimated value and the measured value.

6. (Amended) The information processing system according to any one of claims 1 to 5, wherein

the information acquiring unit performs the acquisition processing for the staying time information and the target information, and

the processing unit performs processing for creating the advice information concerning an exercise amount of the user on the basis of the staying time information, the target information, and the relational expression.

**7.** (Amended) The information processing system according to claim 6, wherein
the information acquiring unit performs the acquisition processing for the staying time information up to given advice timing within an advice period and performs the acquisition processing for acquiring, as the target information, a target value of the body weight or the body fat amount of the user at the advice timing set before the advice timing, and
the processing unit performs processing for calculating an estimated value of the body weight or the body fat amount corresponding to the staying time information on the basis of the staying time information and the relational expression and creating the advice information concerning the exercise amount on the basis of comparison processing of the calculated estimated value and the target value.

**8.** (Amended) The information processing system according to claim 5 or 7, wherein
the information acquiring unit performs the acquisition processing for acquiring, as the body information, a present value of the body weight or the body fat amount of the user at the advice timing and performs the acquisition processing for acquiring, as the target information, a target value of the body weight or the body fact amount of the user and a target period representing a period until the target value is attained, and
the processing unit performs processing for calculating, on the basis of the present value, the target value, the target period, and the relational expression, the staying time information necessary for attaining the target value within the target period.

**9.** (Amended) The information processing system according to claims 1 to 3, wherein
the information acquiring unit performs the acquisition processing for the body information and the target information, and
the processing unit performs processing for calculating, on the basis of the body information, the target information, and the relational expression, the staying time information necessary for attaining a target represented by the target information.

**10.** (Amended) The information processing system according to claim 9, wherein
the information acquiring unit performs the acquisition processing for acquiring a present value of the body weight or the body fat amount of the user as the body information and performs the acquisition processing for acquiring, as the target information, a target value of the body weight or the body fat amount of the user and a target period representing a period until the target value is attained, and
the processing unit performs processing for calculating, on the basis of the present value, the target value, the target period, and the relational expression, the staying time information necessary for attaining the target value within the target period.

**11.** (Amended) The information processing system according to claim 10, wherein
the processing unit performs processing for generating correction instruction information for instructing correction of at least one of the target value and the target period when a staying time represented by the calculated staying time information is larger than a given threshold, and
when the information acquiring unit performs the acquisition processing for at least one of the target value and the target period corrected on the basis of the correction instruction information, the processing unit performs processing for calculating again, on the basis of at least one of the target value and the target period after the correction, the staying time information necessary for attaining the target value within the target period.

**12.** (Amended) The information processing system according to any one of claims 9 to 11, wherein
at start timing of an advice period, the information acquiring unit performs the acquisition processing for the body information and the target information, and
the processing unit performs processing for calculating, on the basis of the body information, the target information, and the relational expression, the staying time information necessary for attaining a target represented by the target information.

**13.** (Amended) A program for causing a computer to function as:

an information acquiring unit that performs acquisition processing for information;
a processing unit that performs processing on the basis of the information acquired by the information acquiring unit; and
an output processing unit that performs output processing for the information created by the processing unit, wherein
the information acquiring unit performs the acquisition processing for at least two kinds of information among staying time information representing a rate of time in which a value represented by pulse wave information of a user is a value within a given fat burning zone per unit time, body information of the user, and target information concerning fluctuation in body weight or a body fat amount of the user,

the processing unit performs processing for creating advice information concerning the fluctuation in the body weight or the body fat amount of the user on the basis of a relational expression representing a relation between the fluctuation in the body weight or the body fat amount of the user and the staying time information and the at least two kinds of information acquired by the information acquiring unit, and

the output processing unit performs the output processing for the advice information created by the processing unit.

**14.** (Added) An information processing method for causing a computer to execute:

acquisition processing for at least two kinds of information among staying time information representing a rate of time in which a value represented by pulse wave information of a user is a value within a given fat burning zone per unit time, body information of the user, and target information concerning fluctuation in body weight or a body fat amount of the user;

processing for creating advice information concerning the fluctuation in the body weight or the body fat amount of the user on the basis of a relational expression representing a relation between the fluctuation in the body weight or the body fat amount of the user and the staying time information and the at least two kinds of information acquired by the acquisition processing; and

the output processing for the created advice information.

**15.** (Added) An information processing system that performs:

acquisition processing for at least two kinds of information among staying time information representing a rate of time in which a value represented by pulse wave information of a user is a value within a given fat burning zone per unit time, body information of the user, and target information concerning fluctuation in body weight or a body fat amount of the user;

processing for creating advice information concerning the fluctuation in the body weight or the body fat amount of the user on the basis of a relational expression representing a relation between the fluctuation in the body weight or the body fat amount of the user and the staying time information and the at least two kinds of information acquired by the acquisition processing; and

the output processing for the created advice information.

**Statement under Art. 19.1 PCT**

Claim 1 after amendment is amended on the basis of claim 1 before amendment and the descriptions of paragraphs [0008] to [0009] of the specification as filed.

Claim 2 added anew is based on the description of paragraph [0071] of the specification as filed.

The characteristics not disclosed in Literatures 1 to 4 cited in the search report are clarified by this amendment.

Claim 3 after amendment is equivalent to claim 2 before amendment.

Claim 4 after amendment is equivalent to claim 3 before amendment.

Claim 5 after amendment is equivalent to claim 4 before amendment.

Claim 6 after amendment is equivalent to claim 5 before amendment.

Claim 7 after amendment is equivalent to claim 6 before amendment.

Claim 8 after amendment is equivalent to claim 7 before amendment.

Claim 9 after amendment is equivalent to claim 8 before amendment.

Claim 10 after amendment is equivalent to claim 9 before amendment.

Claim 11 after amendment is equivalent to claim 10 before amendment.

Claim 12 after amendment is equivalent to claim 11 before amendment.

Claim 13 after amendment is amended on the basis of claim 12 before amendment and the description of paragraph [0071] of the specification as filed. The characteristics not disclosed in Literatures 1 to 4 cited in the search report are clarified by this amendment.

Claim 14 after amendment is amended on the basis of claim 13 before amendment and the description of paragraph [0071] of the specification as filed. The characteristics not disclosed in Literatures 1 to 4 cited in the search report are clarified by this amendment.

Claim 15 added anew is based on claim 13 as filed and the description of paragraph [0071] of the specification as filed.

# FIG. 1

SUBJECT

SUBJECT

MENTOR

PLATFORM (10)

PULSE MACHINE CONNECTION INTERFACE (12)

PLATFORM MAIN BODY (13)

OPERATION MANAGEMENT SERVICE (11)

REPLY

REQUEST

ADVICE ENGINE (15)

REQUEST DATA OF XXX

DATA OF XXX

COMMON DATABASE (USER INFORMATION, PULSE, GPS, BODY COMPOSITION, MEAL RECORD, INFORMATION, ENVIRONMENT, etc) (14)

XML

CSV

WANT ADVICE OF XXX

RETURN ADVICE OF XXX

ADVICE

ADVICE OF XXX

DATA OF XXX

ADVICE

XML

CSV

PREDICTION ENGINE (16)

EP 3 007 127 A1

100

INFORMATION
PROCESSING SYSTEM

ACQUISITION
PROCESSING

110 ～ | INFORMATION
ACQUIRING
UNIT

120 ～ | PROCESSING
UNIT

ADVICE
INFORMATION

130 ～ | OUTPUT
PROCESSING
UNIT

OUTPUT
PROCESSING

# FIG. 2

FIG. 3

FIG. 4

FIG. 5

FIG. 6

ZONE STAYING RATE
(IN-ZONE TIME RATE) (%)

EP 3 007 127 A1

FIG. 7

ZONE STAYING RATE
(IN-ZONE TIME RATE) (%)

FIG. 8

BODY WEIGHT
DECREASE
PER 1 MINUTE

0.00

BODY FAT DECREASE PER 1 MINUTE

EP 3 007 127 A1

(A)

[START TIME]

PRESENT WEIGHT IS 69.8 kg, BMI IS 23.7, AND STANDARD WEIGHT IS 64.8 kg.
THEREFORE, WEIGHT LOSS OF 5 kg IS NECESSARY.
IF YOU PERFORM EXERCISE 2 HOURS IN 1 DAY TO SET PULSE IN ZONE,
YOUR WEIGHT WILL DECREASE TO STANDARD WEIGHT IN 6 MONTHS.
IF YOU FURTHER MAKE EFFORTS THROUGH DIET OR THE LIKE, YOUR
WEIGHT WILL DECREASE TO STANDARD WEIGHT IN 3 MONTHS.

(B)

[AFTER THREE MONTHS]

YOU CONTINUED EXERCISE OF AVERAGE OF 26 MINUTES PER 1 DAY AT
IN-ZONE TIME RATE OF 74% FOR 3 MONTHS.
WEIGHT LOSS OF 0.7 kg CAN BE EXPECTED. ACTUALLY, WEIGHT LOSS
WAS 1.3 kg.
WEIGHT LOSS WAS VERY EXCELLENT. HOWEVER, TAKE CARE NOT TO
PERFORM DIET EXCESSIVELY.
IF YOU WILL CONTINUE EXERCISE OF 2 HOURS 15 MINUTES PER 1 DAY FOR
3 MONTHS AS IN THE PAST, YOUR WEIGHT WILL DECREASE TO STANDARD
WEIGHT.

(C)

[AFTER SIX MONTHS]

YOU CONTINUED EXERCISE OF AVERAGE OF 23 MINUTES PER 1 DAY AT
IN-ZONE TIME RATE OF 76% FOR 6 MONTHS.
WEIGHT LOSS OF 1.3 kg CAN BE EXPECTED. ACTUALLY, WEIGHT LOSS
WAS 0.9 kg.
WEIGHT LOSS WAS VERY EXCELLENT IN FORMER HALF. HOWEVER,
WEIGHT INCREASED IN LATTER HALF. REDUCE INTAKE CALORIE MORE.
IF YOU WILL CONTINUE EXERCISE OF 2 HOURS 25 MINUTES PER 1 DAY FOR
3 MONTHS AS IN THE PAST, YOUR WEIGHT WILL DECREASE TO STANDARD
WEIGHT.

FIG. 9

FIG. 10

# FIG. 11

BODY FAT DECREASE
MEASURED VALUE
PER 1 MINUTE - BODY
FAT DECREASE
PREDICTED VALUE

0.00

0.0

BODY FAT DECREASES MORE
THAN PREDICTED VALUE

BODY FAT DOES NOT DECREASE
MORE THAN PREDICTED VALUE

AVERAGE INTAKE CALORIE OF 1 DAY

# FIG. 12

MEASURED VALUE OF
BODY WEIGHT DECREASE

REGRESSION LINE OF
BODY WEIGHT DEGREASE

C2

PREDICTION LINE OF
BODY WEIGHT DEGREASE

C1

BODY WEIGHT
OR BODY FAT
AMOUNT

ANY PERIOD

NUMBER OF ELAPSED DAYS

# FIG. 13

PREDICTION LINE OF
BODY WEIGHT DEGREASE

D1

BODY WEIGHT
OR BODY FAT
AMOUNT

TARGET LINE OF
WEIGHT LOSS

D2

ANY PERIOD

NUMBER OF ELAPSED DAYS

## FIG. 14

PREDICTED VALUE OF BODY WEIGHT

OLD PREDICTION LINE OF
BODY WEIGHT DEGREASE

NEW PREDICTION LINE OF
BODY WEIGHT DEGREASE

NEW BODY WEIGHT
INITIAL VALUE
(NEW Wini)

NEW START POINT OF T

EVALUATION START DAY

NUMBER OF ELAPSED DAYS

## FIG. 15

BODY WEIGHT OR BODY FAT AMOUNT

REGRESSION LINE OF
BODY WEIGHT DEGREASE

PREDICTION LINE OF BODY
WEIGHT DEGREASE NOT CHANGED

TARGET LINE OF
WEIGHT LOSS

NEW TARGET LINE OF
BODY WEIGHT DECREASE

3 MONTHS          6 MONTHS

NUMBER OF ELAPSED DAYS

FIG. 16

## INTERNATIONAL SEARCH REPORT

| | International application No. |
|---|---|
| | PCT/JP2014/002175 |

**A. CLASSIFICATION OF SUBJECT MATTER**
*G06Q50/22*(2012.01)i

According to International Patent Classification (IPC) or to both national classification and IPC

**B. FIELDS SEARCHED**

Minimum documentation searched (classification system followed by classification symbols)
G06Q50/22

Documentation searched other than minimum documentation to the extent that such documents are included in the fields searched

| Jitsuyo Shinan Koho | 1922–1996 | Jitsuyo Shinan Toroku Koho | 1996–2014 |
|---|---|---|---|
| Kokai Jitsuyo Shinan Koho | 1971–2014 | Toroku Jitsuyo Shinan Koho | 1994–2014 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| Y | JP 2012-501032 A (Koninklijke Philips Electronics N.V.), 12 January 2012 (12.01.2012), paragraphs [0039] to [0042], [0044], [0045], [0047], [0053], [0066] & US 2011/0143322 A1  & EP 2318964 A1 & WO 2010/023593 A1  & CN 102132283 A & KR 10-2011-0073467 A  & TW 201019156 A & RU 2011111280 A | 1-13 |
| Y | JP 2009-240404 A (Citizen Holdings Co., Ltd.), 22 October 2009 (22.10.2009), paragraphs [0002] to [0005] (Family: none) | 1-13 |

| ☒ Further documents are listed in the continuation of Box C. | ☐ See patent family annex. |
|---|---|

| * | Special categories of cited documents: |
|---|---|
| "A" | document defining the general state of the art which is not considered to be of particular relevance |
| "E" | earlier application or patent but published on or after the international filing date |
| "L" | document which may throw doubts on priority claim(s) or which is cited to establish the publication date of another citation or other special reason (as specified) |
| "O" | document referring to an oral disclosure, use, exhibition or other means |
| "P" | document published prior to the international filing date but later than the priority date claimed |

| "T" | later document published after the international filing date or priority date and not in conflict with the application but cited to understand the principle or theory underlying the invention |
|---|---|
| "X" | document of particular relevance; the claimed invention cannot be considered novel or cannot be considered to involve an inventive step when the document is taken alone |
| "Y" | document of particular relevance; the claimed invention cannot be considered to involve an inventive step when the document is combined with one or more other such documents, such combination being obvious to a person skilled in the art |
| "&" | document member of the same patent family |

| Date of the actual completion of the international search | Date of mailing of the international search report |
|---|---|
| 19 May, 2014 (19.05.14) | 27 May, 2014 (27.05.14) |

| Name and mailing address of the ISA/ Japanese Patent Office | Authorized officer |
|---|---|
| Facsimile No. | Telephone No. |

Form PCT/ISA/210 (second sheet) (July 2009)

**INTERNATIONAL SEARCH REPORT**

| International application No. |
| --- |
| PCT/JP2014/002175 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2012-075489 A (Seiko Epson Corp.), 19 April 2012 (19.04.2012), paragraph [0025] & US 2012/0083671 A1 | 1-13 |
| Y | JP 2004-227522 A (Microstone Corp.), 12 August 2004 (12.08.2004), paragraphs [0087] to [0089], [0096], [0143] (Family: none) | 1-13 |

Form PCT/ISA/210 (continuation of second sheet) (July 2009)

**EP 3 007 127 A1**

REFERENCES CITED IN THE DESCRIPTION

**Patent documents cited in the description**

- JP 2008033909 A